(19) Europäisches Patentamt
European Patent Office
Office européen des brevets

(11) EP 1 331 005 B1

(12) EUROPEAN PATENT SPECIFICATION

(45) Date of publication and mention of the grant of the patent:
**05.04.2006 Bulletin 2006/14**

(21) Application number: **01978983.3**

(22) Date of filing: **31.10.2001**

(51) Int Cl.:
***A61K 31/404*** (2006.01) ***A61K 31/4745*** (2006.01)
***A61K 31/407*** (2006.01) ***A61K 31/513*** (2006.01)
***A61K 31/24*** (2006.01) ***A61K 31/7068*** (2006.01)
***A61K 31/704*** (2006.01) ***A61K 31/337*** (2006.01)
***C07D 209/08*** (2006.01) ***C07D 235/06*** (2006.01)
***C07D 231/56*** (2006.01) ***C07D 471/04*** (2006.01)
***A61P 35/00*** (2006.01)

(86) International application number:
**PCT/JP2001/009563**

(87) International publication number:
**WO 2002/036117 (10.05.2002 Gazette 2002/19)**

# (54) MEDICINAL COMPOSITIONS FOR CONCOMINANT USE AS ANTICANCER ATENT

MEDIZINISCHE ZUSAMMENSETZUNGEN ALS BEGLEITTHERAPIE GEGEN KREBS

COMPOSITIONS MEDICINALES DESTINEES A ETRE UTILISE DE MANIERE CONCOMITANTE COMME AGENT ANTICANCEREUX

(84) Designated Contracting States:
**AT BE CH CY DE DK ES FI FR GB GR IE IT LI LU MC NL PT SE TR**

(30) Priority: **31.10.2000 JP 2000333952**

(43) Date of publication of application:
**30.07.2003 Bulletin 2003/31**

(73) Proprietor: **Eisai Co., Ltd.**
**Tokyo 112-8088 (JP)**

(72) Inventors:
- **OZAWA, Yoichi**
**Tsukuba-shi, Ibaraki 305-0051 (JP)**
- **YOSHIMATSU, Kentaro**
**Tsuchiura-shi, Ibaraki 300-0845 (JP)**
- **KAI, Junko**
**Niihari-gun, Ibaraki 300-4118 (JP)**

(74) Representative: **HOFFMANN EITLE**
**Patent- und Rechtsanwälte**
**Arabellastrasse 4**
**81925 München (DE)**

(56) References cited:
**EP-A- 0 673 937** **WO-A1-00/50395**
**JP-A- 9 208 571** **JP-A- 2000 143 635**

- **MILLER V A, RIGAS J R, GRANT S C, PISTERS K M W, KRIS M G: "New Chemotherapeutic Agents for Non-small Cell Lung Cancer" CHEST, vol. 107, no. 6 suppl, 1995, pages 306S-311S, XP002302019**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

EP 1 331 005 B1

## Description

### Technical Field

The present invention relates to a novel medicinal composition comprising a compound useful as an anticancer drug, particularly N-(3-chloro-1H-indol-7-yl)-4-sulfamoylbenzenesulfonamide or a pharmacologically acceptable salt thereof, combined with another anticancer agent.

### Prior Art

As chemicals used conventionally as chemotherapeutic drugs for cancers, there are a large number of chemicals such as cyclophosphamide as an alkylating agent, methotrexate and fluorouracil as antimetabolites, doxorubicin, mitomycin and bleomycin as antibiotics, vincristine and etoposide derived from plant, and cisplatin as a metal complex, but their antitumor effects are insufficient, so there is a demand for development of new antitumor agents.

On one hand, the present inventors succeeded for the first time in synthesis of completely new aromatic sulfonamides or aromatic sulfonate compounds, they unexpectedly found that these compounds exhibit an excellent antitumor activity, and they provided useful antitumor agents showing new mechanism of action (JP-A 7-165708). In particular, N-(3-chloro-1H-indol-7-yl)-4-sulfamoylbenzenesulfonamide (hereinafter, also called "E7070") represented by the following formula (II):

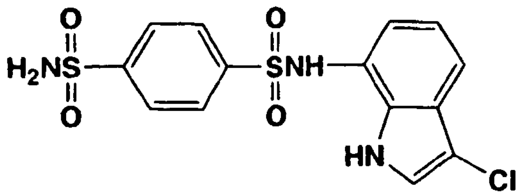

(II)

exhibits an activity on various types of tumors and is very useful.

There is the case where a certain kind of compound is effective against a certain kind of cancer but poor in effectiveness against other cancers. Further, administration of a single component may not achieve a sufficient effect. That is, the object of the present invention is to provide a medicinal composition having an excellent antitumor activity capable of solving these problems.

### Disclosure of the Invention

In view of the foregoing, the present inventors made extensive study to search for an excellent antitumor composition, and as a result, they unexpectedly found that a synergistically further excellent antitumor activity can be achieved by using a certain anticancer agent in combination with a sulfonamide compound, a sulfonate compound or a salt of them (sulfone compound (I)), which is represented by the following formula:

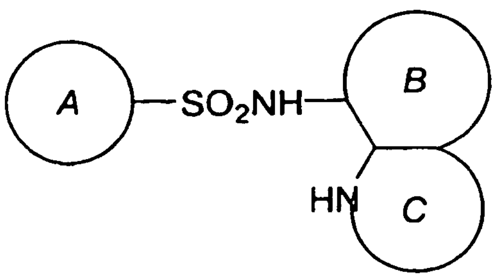

(I)

wherein

ring *A* is benzene or pyridine each optionally substituted with 1-3 groups independently selected from amino, ($C_{1-6}$-alkyl)amino, ($C_{3-8}$-cycloalkyl) amino, $C_{1-6}$-alkyl, $C_{1-6}$-alkoxy, hydroxyl, nitro, mercapto, cyano, $C_{1-6}$-alkylthio, halogen, and groups selected from the formulae (i)-(vi):

(i) $-a-CH_2-d$
(ii) -a-e-f
(iii) -a-g-h
(iv) $-a-N(R^6)-g-i$
(v) $-a-N(R^7)-e-f$
(vi) $-(CH_2)_p-j-(CH_2)_q-d$

wherein
a is a single bond or $-Z-(CH_2)_k-$;
d is amino, ($C_{1-6}$-alkyl)amino, halogen, hydroxyl, $C_{1-6}$-alkylthio, cyano or $C_{1-6}$-alkoxy;
e is -SO- or $-SO_2-$;
f is amino, ($C_{1-6}$-alkyl)amino, ($C_{1-6}$-alkoxy)amino, $C_{1-6}$-alkyl, trifluoromethyl, $-(CH_2)_m-d$ or $-N(R^4)-(CH_2)_m-d$;
g is -CO- or -CS-;
h is amino, hydroxyl, $C_{1-6}$-alkyl, $C_{1-6}$-alkoxy, - $(CH_2)_n-d$ or $-N(R^5)-(CH_2)_n-d$ (wherein d is as defined above) ;
i is H, $C_{1-6}$-alkoxy or f as defined above;
j is O or S;
Z is a bond, O, S or $NR^3$;$R^3$, $R^4$, $R^5$, $R^6$ and $R^7$ are each H or $C_{1-6}$-alkyl; k and p are each an integer of 1-5; and m, n and q are each an integer of 2-6;

ring *B* is optionally substituted benzene; and
ring *C* is optionally substituted pyrrole; rings *B* and *C* each individually may be substituted with 1-2 groups independently selected from halogen, cyano, $C_{1-6}$-alkyl, $C_{1-6}$-alkoxy, hydroxyl, oxo, amino, ($C_{1-6}$-alkyl)amino, trifluoromethyl, and -CO-r (wherein r is selected from H, amino, ($C_{1-6}$-alkyl)amino, $C_{1-6}$alkyl, $C_{1-6}$-alkoxy and hydroxyl);

and particularly with N-(3-chloro-1H-indol-7-yl)-4-sulfamoyl benzenesulfonamide (E7070) or a salt thereof, and the present invention was thereby completed.

That is, the present invention relates to:

(1) a pharmaceutical composition comprising the above mentioned compound (I), combined with at least one compound (III) selected from irinotecan hydrochloride trihydrate, cisplatin; mitomycin C, 5-fluorouracil; gemcitabine hydrochloride; taxol; doxorubicin; and salts thereof.
(2) The pharmaceutical composition described in the above (1), comprising, as a compound of formula (I), N-(3-chloro-1H-indol-7-yl)-4- sulfamoylbenzenesulfonamide or a salt thereof.
(3) The pharmaceutical composition described in the above (2), which comprises, as a compound (III), irinotecan hydrochloride trihydrate or a salt thereof.
(4) The pharmaceutical composition described in the above (2), which comprises, as a compound (III), mitomycin C or a salt thereof.
(5) The pharmaceutical composition described in the above (2), which comprises, as a compound (III), 5-fluorouracil or a salt thereof.
(6) The pharmaceutical composition described in the above (2), which comprises, as a compound (III), cisplatin or a salt thereof.
(7) The pharmaceutical composition described in the above (2), which comprises, as a compound (III), irinotecan hydrochloride trihydrate or a salt thereof, and cisplatin or a salt thereof.
(8) The pharmaceutical composition described in the above (7), which further comprises 5-fluorouracil or a salt thereof.
(9) The pharmaceutical composition described in any of the above (1)-(8) wherein the sulfone compound (I) and the substance (III) are present for simultaneous or separate administration to a patient.
(10) The use of a combination of (i) a compound of formula (I) or a salt thereof as described in the above (1), and (ii) at least one compound (III) selected from irinotecan hydrochloride trihydrate; mitomycin C; cisplatin; 5-fluorouracil; gemcitabine hydrochloride; taxol; doxorubicin; and salts thereof, for producing a medicament effective in the prevention or treatment of cancer.
(11) The use described in the above (10), wherein the compound of formula (I) is N-(3-chloro-1H-indol-7-yl)-4-sulfamoylbenzenesulfonamide or a salt thereof.
(12) The use described in the above (10) or (11), wherein the medicament is for simultaneous or separate administration of the sulfone compound (I) and the substance (III) to a patient.

The pharmaceutical composition and the use according to the present invention can be practiced respectively in methods described later.

Hereinafter, the meanings of symbols, terms etc. used in this specification are described, and the present invention is described in detail.

In the specification of this application, although structural formula of a compound may express a certain isomer for the sake of convenience, it goes without saying that the compounds contained in the medicinal composition according to the present invention are not limited to those of the structural formulae shown for convenience's sake and all possible isomers which can occur in the structures, for example geometric isomer, optical isomer based on asymmetrical carbon, rotational isomer, stereoisomer and tautomer, as well as a mixture of such isomers are included. Further, as the above-mentioned compound, crystal polymorphism may be present but, again, there is no limitation but any of single crystal form or a mixture will do. Further, the above-mentioned compound may be an anhydride or a hydrate, and either of them are included in the scope of claim for patent in the present invention. The medicinal composition according to the present invention exhibits a strong antitumor activity, and it also includes derivatives of the above-mentioned, which exhibit an antitumor activity as a result of metabolism such as oxidation, reduction, hydrolysis and conjugation in vivo. Further, the present invention also includes the compounds which produce the compound in the composition of the present invention as a result of metabolism such as oxidation, reduction and hydrolysis *in vivo.*

The ring A in Formula (I) is selected from benzene and pyridine.

In this specification, the ring B refers to a benzene ring.

In this specification, the ring C refers to pyrrole.

The ring A may be substituted with 1 to 3 substituent groups, and when a plurality of substituent groups are present, the definitions of the substituent groups are independent. The "substituent group" includes (1) an amino group which may be substituted with a $C_{1-6}$-alkyl group or a $C_{3-8}$ cycloalkyl group, (2) a $C_{1-6}$ alkyl group, (3) a $C_{1-6}$-alkoxy group, (4) hydroxyl, (5) nitro (6) mercapto, (7) cyano, (8) a $C_{1-6}$-alkylthio group, (9) halogen, (10) -a-b (wherein a is a single bond, $-(CH_2)_k-$, $-O-(CH_2)_k-$, $-S-(CH_2)_k-$ or $-N(R^3)-(CH_2)_k-$ (wherein k is an integer of 1 to 5; and $R^3$ represents hydrogen atom or a $C_{1-6}$-alkyl group ; and b is $-CH_2$-d (wherein d represents an amino group which may be substituted with a : alkyl group, or halogen, hydroxyl, a $C_{1-6}$ alkylthio group, cyano or a $C_{1-6}$-alkoxy group carbon atoms)), (11) -a-e-f (wherein a has the same meaning as defined above; e represents -SO- or $-SO_2-$; and f represents an amino group which may be substituted with a $C_{1-6}$-alkyl group or a $C_{1-6}$-alkoxy group, a $C_{1-6}$-alkyl group, trifluoromethyl group, $-(CH_2)_m$-b or $-N(R^4)-(CH_2)_m$-b (wherein b has the same meaning as defined above; $R^4$ represents a hydrogen atom or a $C_{1-6}$-alkyl group; and m is an integer of 1 to 5)), (12) -a-g-h (wherein a has the same meaning as defined above; g represents -CO- or -CS-; and h represents an optionally substituted amino group, hydroxyl, a $C_{1-6}$-alkyl group, a $C_{1-6}$-alkoxy group, $-(CH_2)_n$-b or $-N(R^5)-(CH_2)_n$-b (wherein b has the same meaning as defined above; $R^5$ represents hydrogen or a $C_{1-6}$-alkyl group and n is an integer of 1 to 5)), (13) $-a-N(R^6)$-g-i (wherein a and g have the same meanings as defined above; $R^6$ represents hydrogen or a $C_{1-6}$-alkyl group; i represents hydrogen, a $C_{1-6}$-alkoxy group or f (wherein f has the same meaning as defined above)), (14) $-a-N(R^7)$-e-f (wherein a, e and f have the same meanings as defined above; and $R^7$ represents hydrogen or a $C_{1-6}$-alkyl group), and (15)-$(CH_2)_p$-j-$(CH_2)_q$-b (wherein j is o or S; b has the same meaning as defined above; and p and q independently represent an integer of 1 to 5).

When an amino group in the substituent group is substituted with 2 alkyl groups, the alkyl groups may be combined together via a linkage to form a 5- or 6-membered nitrogen-containing ring.

When the ring A is a pyridine ring having hydroxyl group or mercapto group as a substituent group, the substituent group may be an oxo or thioxo group by a resonance structure.

The rings B and C may each individually be substituted with 1 or 2 substituent groups, and when a plurality of substituent groups are present, the definitions of the substituent groups are independent. The substituent groups on the rings B and C are also independent.

The "substituent group" includes halogen, cyano, a $C_{1-6}$-alkyl group, a $C_{1-6}$-alkoxy group, hydroxyl, oxo, -CO-r (wherein r represents hydrogen, an amino group which may be substituted with a $C_{1-6}$-alkyl group, a $C_{1-6}$-alkyl group, a $C_{1-6}$-alkoxy group or hydroxyl), an amino group which may be substituted with a $C_{1-6}$-alkyl group and trifluoromethyl group.

The "$C_{1-6}$-akyl group" used in this specification refers to an alkyl group containing 1 to 6 carbon atoms, and preferable examples include linear or branched alkyl groups such as methyl, ethyl, n-propyl, iso-propyl, n-butyl, iso-butyl, sec-butyl, tert-butyl, n-pentyl, 1.1-dimethylpropyl, 1,2-dimethylpropyl, 2,2-dimethylpropyl, 1-ethylpropyl, 2-ethylpropyl, n-hexyl, 1-methyl-2-ethylpropyl, 1-ethyl-2-methylpropyl, 1,1,2-trimethylpropyl, 1-propylpropyl, 1-methylbutyl , 2-methylbutyl, 1,1-dimethylbutyl, 1,2-dimethylbutyl, 2,2-dimethylbutyl 1,3-dimethylbutyl, 2,3-dimethylbutyl, 2-ethylbutyl, 2-methylpentyl and 3-methylpentyl, more preferably methyl, ethyl, n-propyl, iso-propyl, n-butyl and iso-butyl, still more preferably methyl, ethyl group, n-propyl and iso-propyl.

The "$C_{3-8}$-cycloalkyl group" used in this specification refers to a cycloalkyl group composed of 3 to 8 carbon atoms, and preferable examples include cyclopropyl, cyclobutyl, cylopentyl, cyclohexyl, cycloheptyl and cyclooctyl.

The "$C_{1-6}$ alkoxy group" used in this specification refers to an alkoxy group containing 1 to 6 carbon groups, and preferable examples thereof include methoxy, ethoxy n-propoxy, iso-propoxy, sec-propoxy, n-butoxy, iso-butoxy, sec-butoxy, tert-butoxy, n-pentyloxy, iso-pentylox, sec-pentyloxy, n-hexoxy, iso-hexoxy, 1,1-dimethyl propyloxy,

1,2-dimethyl propoxy, 2,2-dimethyl propyloxy, 2-ethyl propoxy, 1-methyl-2-ethyl propoxy, 1-ethyl-2-methylpropoxy, 1,1,2-trimethylpropoxy, 1,1,2-trimethylpropoxy 1,1-dimethylbutoxy, 1,2-dimethylbutoxy, 2,2-dimethylbutoxy, 2,3-dimethylbutyloxy 1,3-dimethylbutyloxy, 2-ethylbutoxy, 1,3-dimethylbutoxy, 2-methylpentoxy, 3-methylpentoxy and hexyloxy, most preferably methoxy and ethoxy.

The "halogen" used in this specification refers to a halogen atom, and includes fluorine chlorine, bromine, and iodine, and preferable examples include fluorine, chlorine and bromine.

Preferable examples of the compound (I) contained in the medicinal composition according to the present invention are not particularly limited, but the most preferable compound is N-(3-chloro-1H-indol-7-yl)-4-sulfamoylbenzenesulfonamide or a pharmacologically acceptable salt thereof.

In this specification, N-(3-chloro-1H-indol-7-yl)-4-sulfamoylbenzenesulfonamide and E7070 refer to a compound represented by the formula (II):

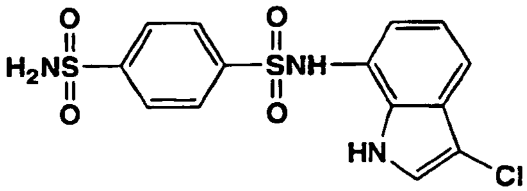

(II)

Irinotecan hydrochloride trihydrate (CPT-11; [1,4'-Bipiperidine]-1'-carboxylic acid (S)-4,11-diethyl-3,4,12,14-tetrahydro-4-hydroxy-3,19-dioxo-1H-pyrano-[3',4' :6,7]-indolizino[1,2-b]quinolin-9-yl ester Hydrochloride trihydrate) in this specification refers to a compound represented by the formula:

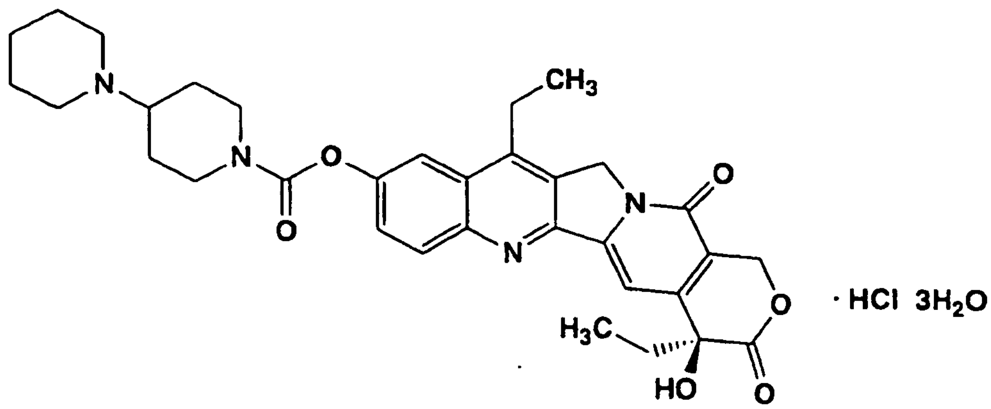

Mitomycin C (MMC; [1aS-(1aα,8β,8aα, 8bα)]-6-amino-8-[[(aminocarbonyl)oxy]methyl]1,1a,2,8,8a,8b-hexahydro-8a-methoxy-5-methylazirino[2',3':3,4]pyrrolo[1,2-a]indole-4,7-dione)) in this specification refers to a compound represented by the formula:

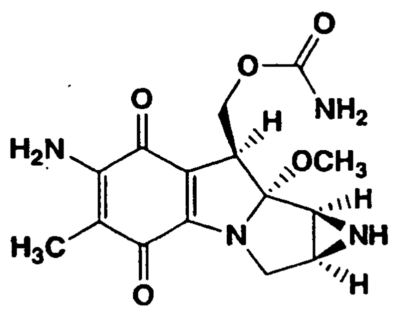

5-Fluorouracil (5-FU (5-fluoro-2,4-(1H,3H)-pyrimidinedione)) in this specification refers to a compound represented by the formula:

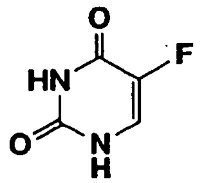

Cisplatin (CDDP (cis-diamminedichloroplatinum)) in this specification refers to a compound represented by the formula:

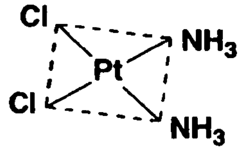

Gemcitabine hydrochloride (2'-deoxy-2',2'-difluorocytidine hydrochloride) in this specification refers to a compound represented by the formula:

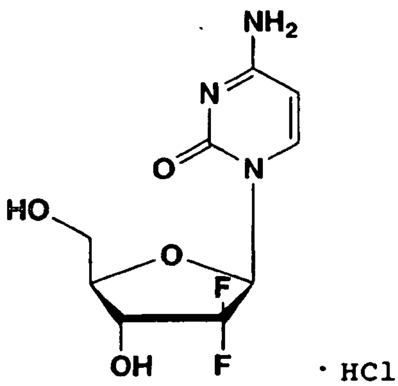

Doxorubicin (adryamicin; (10-[(3-amino-2,3,6-trideoxy-α-L-lyxo-hexopyranosyl)oxy]-7,8,9,10-tetrahydro-6,8,11-trihydroxy-8-(hydroxyacetyl)-1-methoxy-(8S-cis)-5,12-naphthacenedione) in this specification refers to a compound represented by the formula:

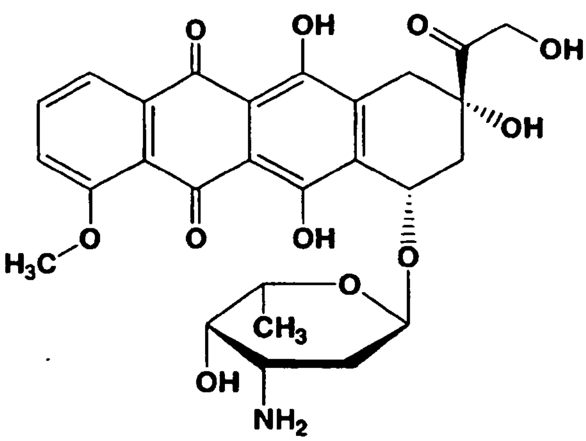

Taxol (paclitaxel; (2R, 5R, 7S, 10R, 13S)-10, 20-bis(acetoxy)-2-benzoyloxy-1,7-dihydroxy-9-oxo-5,20-epoxytax-11-en-13-yl (3S)-3-benzoylamino-3-phenyl-D-lactose) in this specification refers to a compound represented by

the formula:

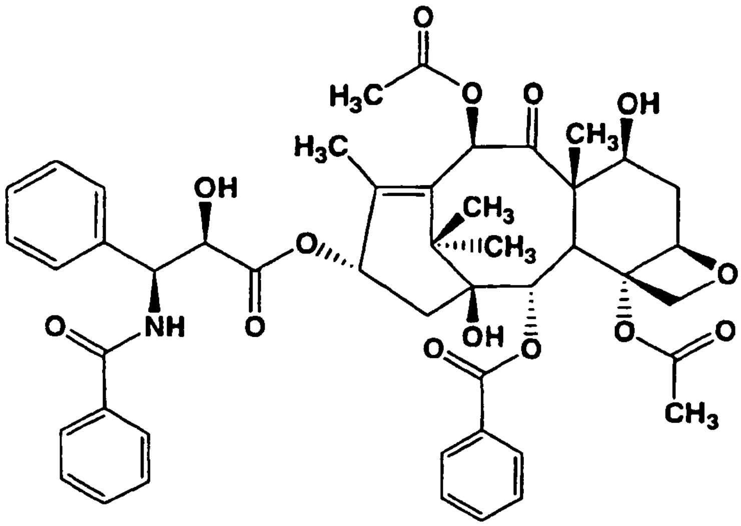

The type of the "salts" used in this specification is not particularly limited, and examples thereof include additive salts of inorganic acids such as hydrochloride, sulfate, carbonate, bicarbonate, hydrobromate and hydroiodate; additive salts of organic carboxylic acids such as acetate, maleate, lactate, tartarate and trifluoroacetate; additive salts of organic sulfonic acid such as methanesulfonate, hydroxymethanesulfonate, hydroxyethanesulfonate, benzenesulfonate, toluenesulfonate and taurine salt; additive salts of amines such as trimethylamine salt, triethylamine salt, pyridine salt, procaine salt, picoline salt, dicyclohexylamine salt, N,N'-dibenzylethylene diamine salt, N-methylglucamine salt, diethanolamine salt, triethanolamine salt, tris(hydroymethylamino)methane salt, and phenethylbenzylamine salt ; and additive salts of amino acids such as arginine salt, lysine salt, serine salt, glycine salt, aspartate and glutamate.

The above-mentioned compound (I) can be produced according to the methods described in e.g. JP-A 7-165708, WO95/07276 etc., or its analogous method.

N-(3-Chloro-1H-indol-7-yl)-4-sulfamoylbenzenesulfonamide (E7070) can be synthesized according to a known method (WO95/07276 or Example 19 in JP-A 7-165708) or its analogous method.

CPT-11, mitomycin C, 5-fluorouracil, cisplatin, gemcitabine hydrochloride, doxorubicin and taxol are known compounds and can be produced in known methods or their analogous methods.

The starting compound and various reagents in the production processes may have formed salts or hydrates which vary depending on the starting material, the solvent used etc. , and are not particularly limited so long as the reaction is not inhibited. Also, the solvent used varies depending on the starting material, reagents etc., and is not particularly limited insofar as it is inert to the reaction and dissolves the starting material to a certain degree. When the desired compound is obtained in a free form, it can be converted in a usual manner into a pharmaceutically acceptable salt. Further, the resultant various isomers (for example, geometric isomer, optical isomer based on asymmetric carbon, rotational isomer, stereoisomer and tautomer) can be purified and isolated by usual separating means, for example, re-crystallization, diastereomer salt method, enzyme resolution method, and various kinds of chromatography (for example, thin-layer chromatography, column chromatography and gas chromatography).

The "anticancer drug" in this specification means a drug used against tumors, particularly malignant .tumors. The cancer disease against which the medicinal composition or the anticancer drug according to the present invention is effective includes, for example, brain cancer, head and neck cancer, cancer of the esophagus, cancer of the stomach, cancer of the colon, hepatoma, pancreatic cancer, lung cancer, breast cancer, skin cancer, ovarian cancer, prostatic cancer, renal cancer, bladder cancer, lymphoma and leukemia. The medicinal composition or the anticancer drug according to the present invention is useful for treating or preventing cancer diseases in mammalians (e.g., humans, mice, rats, guinea pigs, rabbits, dogs, horses and monkeys), particularly for treating or preventing cancer diseases in humans.

In the present invention, "the medicinal composition ..., combined with" and "the medicinal composition comprising" refer respectively to a "medicinal composition" comprising two or more active ingredients or medicinal compositions combined therein, and the "medicinal composition" may be prepared and administered as a single preparation comprising two or more active ingredients, or two or more medicinal compositions are separately prepared and simultaneously administered, or two or more medicinal compositions are separately prepared, and one of the compositions is administered, and after a predetermined time, the other composition may be administered. The "medicinal composition" in this invention encompasses any medicinal compositions in these administration forms. Further, the proportion of

preferable ingredients in two or more medicinal compositions to be combined is not particularly limited and may be suitably selected. The proportion of the sulfone compound (I) and the substance (III) in the combined preparation is selected such that on the basis of the total dose for 3 weeks in clinical use, 1% by weight of the sulfone compound (I) such as E7070 or a salt thereof is combined with 0.26 to 1.0% by weight of irinotecan hydrochloride trihydrate or a salt thereof, 0.017 to 0.068% by weight of mitomycin C or a salt thereof, 3.7 to 15% by weight of 5-fluorouracil or a salt thereof, 0.071 to 0.29% by weight of cisplatin or a salt thereof, 2.1 to 8.6% by weight of gemcitabine hydrochloride or a salt thereof, 0.042 to 0.17% by weight of doxorubicin or a salt thereof (preferably hydrochloride), or 0.15 to 0. 60% by weight of taxol or a salt thereof.

One preferable example of the "medicinal composition" according to the present invention is a medicinal composition comprising N-(3-chloro-1H-indol-7-yl)-4-sulfamoylbenzenesulfonamide (E7070) or a salt thereof, combined with at least one substance selected from (1) irinotecan hydrochloride trihydrate; (2) mitomycin C; (3) 5-fluorouracil; (4) cisplatin; (5) gemcitabine hydrochloride; (6) doxorubicin; (7) taxol; and (8) a salt of the above-mentioned (1) to (7), and more preferable examples include the following medicinal compositions:

(a) a medicinal composition comprising E7070 or a salt thereof, combined with irinotecan hydrochloride trihydrate or a salt thereof;
(b) a medicinal composition comprising E7070 or a salt thereof, combined with mitomycin C or a salt thereof;
(c) a medicinal composition comprising E7070 or a salt thereof, combined with 5-fluorouracil or a salt thereof;
(d) a medicinal composition comprising E7070 or a salt thereof, combined with cisplatin or a salt thereof;
(e) a medicinal composition comprising E7070 or a salt thereof, combined with gemcitabine hydrochloride or a salt thereof;
(f) a medicinal composition comprising E7070 or a salt thereof, combined with doxorubicin or a salt thereof;
(g) a medicinal composition comprising E7070 or a salt thereof, combined with taxol or a salt thereof;
(h) a medicinal composition comprising E7070 or a salt thereof, combined with irinotecan hydrochloride trihydrate or a salt thereof and mitomycin C or a salt thereof;
(i) a medicinal composition comprising E7070 or a salt thereof, combined with irinotecan hydrochloride trihydrate or a salt thereof and 5-fluorouracil or salt thereof;
(j) a medicinal composition comprising E7070 or a salt thereof, combined with irinotecan hydrochloride trihydrate or a salt thereof and cisplatin or a salt thereof;
(k) a medicinal composition comprising E7070 or a salt thereof, combined with mitomycin C or a salt thereof and 5-fluorouracil or a salt thereof;
(l) a medicinal composition comprising E7070 or a salt thereof, combined with mitomycin C or a salt thereof and cisplatin or a salt thereof;
(m) a medicinal composition comprising E7070 or a salt thereof, combined with 5-fluorouracil or a salt thereof and cisplatin or a salt thereof;
(n) a medicinal composition comprising E7070 or a salt thereof, combined with mitomycin C or a salt thereof, 5-fluorouracil or a salt thereof and cisplatin or a salt thereof;
(o) a medicinal composition comprising E7070 or a salt thereof, combined with irinotecan trihydrate or a salt thereof, 5-fluorouracil or a salt thereof and cisplatin or a salt thereof;
(p) a medicinal composition comprising E7070 or a salt thereof, combined with irinotecan hydrochloride trihydrate or a salt thereof, mitomycin C or a salt thereof and cisplatin or a salt thereof;
(q) a medicinal composition comprising E7070 or a salt thereof, combined with irinotecan hydrochloride trihydrate or a salt thereof, mitomycin C or a salt thereof and 5-fluorouracil or a salt thereof;
(r) a medicinal composition comprising E7070 or a salt thereof, combined with irinotecan hydrochloride trihydrate or a salt thereof, mitomycin C or a salt thereof, 5-fluorouracil or a salt thereof and cisplatin or a salt thereof;
(s) a medicinal composition comprising E7070 or a salt thereof, combined with irinotecan hydrochloride trihydrate or a salt thereof and gemcitabine hydrochloride or a salt thereof;
(t) a medicinal composition comprising E7070 or a salt thereof, combined with irinotecan hydrochloride trihydrate or a salt thereof and doxorubicin or a salt thereof;
(u) a medicinal composition comprising E7070 or a salt thereof, combined with irinotecan hydrochloride trihydrate or a salt thereof and taxol or a salt thereof;
(v) a medicinal composition comprising E7070 or a salt thereof, combined with mitomycin C or a salt thereof and gemcitabine hydrochloride or a salt thereof;
(w) a medicinal composition comprising E7070 or a salt thereof, combined with mitomycin C or a salt thereof and doxorubicin or a salt thereof;
(x) a medicinal composition comprising E7070 or a salt thereof, combined with mitomycin C or a salt thereof and taxol or a salt thereof;
(y) a medicinal composition comprising E7070 or a salt thereof, combined with 5-fluorouracil or a salt thereof and

gemcitabine hydrochloride or a salt thereof;

(z) a medicinal composition comprising E7070 or a salt thereof, combined with 5-fluorouracil or a salt thereof and doxorubicin or a salt thereof;

(aa) a medicinal composition comprising E7070 or a salt thereof, combined with 5-fluorouracil or a salt thereof and taxol or a salt thereof;

(bb) a medicinal composition comprising E7070 or a salt thereof, combined with cisplatin or a salt thereof and gemcitabine hydrochloride or a salt thereof;

(cc) a medicinal composition comprising E7070 or a salt thereof, combined with cisplatin or a salt thereof and doxorubicin or a salt thereof;

(dd) a medicinal composition comprising E7070 or a salt thereof, combined with cisplatin or a salt thereof and taxol or a salt thereof;

(ee) a medicinal composition comprising E7070 or a salt thereof, combined with gemcitabine hydrochloride or a salt thereof and doxorubicin or a salt thereof;

(ff) a medicinal composition comprising E7070 or a salt thereof, combined with gemcitabine hydrochloride or a salt thereof and taxol or a salt thereof;

(gg) a medicinal composition comprising E7070 or a salt thereof, combined with doxorubicin or a salt thereof and taxol or a salt thereof;

(ee) a medicinal composition comprising E7070 or a salt thereof, combined with gemcitabine hydrochloride or a salt thereof and doxorubicin or a salt thereof;

(ff) a medicinal composition comprising E7070 or a salt thereof, combined with gemcitabine hydrochloride or a salt thereof and taxol or a salt thereof;

(gg) a medicinal composition comprising E7070 or a salt thereof, combined with doxorubicin or a salt thereof and taxol or a salt thereof;

(hh) a medicinal composition comprising E7070 or a salt thereof, combined with mitomycin C or a salt thereof and 5-fluorouracil or a salt thereof and cisplatin or a salt thereof;

(ii) a medicinal composition comprising E7070 or a salt thereof, combined with mitomycin C or a salt thereof, 5-fluorouracil or a salt thereof and gemcitabine hydrochloride or a salt thereof;

(jj) a medicinal composition comprising E7070 or a salt thereof, combined with mitomycin C or a salt thereof, 5-fluorouracil or a salt thereof and doxorubicin and a salt thereof;

(kk) a medicinal composition comprising E7070 or a salt thereof, combined with mitomycin C or a salt thereof, 5-fluorouracil or a salt thereof and taxol or a salt thereof;

(ll) a medicinal composition comprising E7070 or a salt thereof, combined with 5-fluorouracil or a salt thereof, cisplatin or a salt thereof and gemcitabine hydrochloride or a salt thereof;

(mm) a medicinal composition comprising E7070 or a salt thereof, combined with 5-fluorouracil or a salt thereof, cisplatin or a salt thereof and doxorubicin or a salt thereof;

(nn) a medicinal composition comprising E7070 or a salt thereof, combined with 5-fluorouracil or a salt thereof, cisplatin or a salt thereof and taxol or a salt thereof;

(oo) a medicinal composition comprising E7070 or a salt thereof, combined with cisplatin or a salt thereof, gemcitabine hydrochloride or a salt thereof and doxorubicin or a salt thereof;

(pp) a medicinal composition comprising E7070 or a salt thereof, combined with cisplatin or a salt thereof, gemcitabine hydrochloride or a salt thereof and taxol or a salt thereof; and

(qq) a medicinal composition comprising E7070 or a salt thereof, combined with gemcitabine hydrochloride or a salt thereof, doxorubicin or a salt thereof and taxol or a salt thereof.

The ingredients described above can be used to constitute the medicinal composition of the invention, and for administration of the medicinal composition or the anticancer drug, the administration is not limited to simultaneous administration, and the respective ingredients can be administrated separately at predetermined intervals to increase their synergistic effect.

Specifically, the synergistic effect can be increased by a method for preventing or treating cancers, by administering E7070 or a salt thereof to a patient; and administering, after a predetermined time, at least one substance selected from (1) irinotecan hydrochloride trihydrate; (2) mitomycin C; (3) 5-fluorouracil; (4) cisplatin; (5) gemcitabine hydrochloride; (6) doxorubicin; (7) taxol; and (8) a salt of the above-mentioned (1) to (7) to the patient, or by a method for preventing or treating cancers, by administering at least one substance selected from (1) irinotecan hydrochloride trihydrate; (2) mitomycin C; (3) 5-fluorouracil; (4) cisplatin; (5) gemcitabine hydrochloride; (6) doxorubicin; (7) taxol; and (8) a salt of the above-mentioned (1) to (7) to a patient; and administering, after a predetermined time, E7070 or a salt thereof to the patient.

The synergistic antitumor effect can also be achieved by using a pharmaceutical kit for administering effective amounts of E7070 or a salt thereof and at least one substance selected from (1) irinotecan hydrochloride trihydrate; (2)

mitomycin C; (3) 5-fluorouracil; (4) cisplatin; (5) gemcitabine hydrochloride; (6) doxorubicin; (7) taxol; and (8) a salt of the above-mentioned (1) to (7) simultaneously or separately to a patient, for the purpose of administration of the respective ingredients at predetermined intervals as in the above-mentioned methods for preventing or treating. For example, mention is made of a pharmaceutical kit wherein the ingredients, each being packed in a small vessel, are packed in one box.

When the medicinal composition of the present invention is used as a pharmaceutical preparation, the administration form is not particularly limited, and it is administered orally or parenterally. It is useful for treatment and prevention in mammalians (e.g., humans, mice, rats, guinea pigs, rabbits, dogs, horses and monkeys), particularly in humans. The administration dose varies depending on the severity of symptoms, the age, sex, body weight and chemical sensitivity of the patient, administration form, administration time, administration intervals, the properties, prescription and type of the pharmaceutical preparation, and the type of active ingredient, and is not particularly limited. For example, E7070 or a salt thereof is given daily in one portion or in divided portions into a man in a dose of usually about 5 to 6000 mg, preferably about 50 to 4000 mg, more preferably 100 to 3000 mg. The daily doses of the other ingredients are usually as follows:

irinotecan hydrochloride trihydrate, 40 to 150 mg/$m^2$; mitomycin C, 2 to 40 mg; 5-fluorouracil, 5 to 20 mg/kg; cisplatin, 10 to 90 mg/$m^2$; gemcitabine hydrochloride, 500 to 1200 mg/$m^2$; doxorubicin hydrochloride, 10 to 60 mg; and taxol, 135 to 210 mg/$m^2$, but these are standard doses in the case of administering a single drug. In the present invention, the dose can be suitably changed depending on the constitution of the ingredient. For example, the daily dose of the respective ingredients for an adult can be determined usually in the range of 1 to 6000 mg, preferably about 10 to 1000 mg, more preferably 20 to 300 mg.

The medicinal composition according to the invention can be prepared by using the active ingredients as they are or mixing them with pharmacologically acceptable carriers known per se by a conventional method. Preferable preparation forms include injections, tablets, powders, fine granules, granules, coated tablets, capsules, syrups, troches, inhalations, suppositories, ointments, eye ointments, eye drops, nose drops, ear drops, poultices and lotions. To prepare these pharmaceutical preparations, ordinarily used fillers, binders, lubricants, coloring agents, flavoring agents and, if necessary, stabilizers, emulsifiers, absorption promoters and surfactants can be used. Ingredients used generally as starting materials for pharmaceutical preparations can be blended in a usual manner for manufacturing.

As the above-mentioned preparation components, for example, include animal or vegetable oils such as soybean oil, beef tallow or synthetic glyceride; hydrocarbons such as liquid paraffin, squalane or solid paraffin; ester oils such as octyldodecyl myristate or isopropyl myristate; higher alcohols such as cetostearyl alcohol or behenyl alcohol; silicon resin; silicon oil; surfactants such as polyoxyethylene fatty acid ester, sorbitan fatty acid ester, glycerol fatty acid ester, polyoxyethylenesorbitan fatty acid ester, polyoxyethylene hydrogenated castor oil or polyoxyethylene/polyoxypropylene block copolymers; water-soluble polymers such as hydroxyethyl cellulose, polyacrylic acid, carboxyvinyl polymers, polyethylene glycol, polyvinylpyrrolidone or methyl cellulose; alcohols such as ethanol or isopropanol; polyhydric alcohols such as glycerol, propylene glycol, dipropylene glycol or sorbitol; sugars such as glucose or sucrose; inorganic powders such as silicic anhydride, aluminum magnesium silicate or aluminum silicate; and purified water. For pH adjustment, inorganic acids such as hydrochloric acid or phosphoric acid, alkali metal salts of inorganic acids such as sodium phosphate, inorganic bases such as sodium hydroxide, organic acids such as lower fatty acids, citric acid or lactic acid, alkali metal salts of organic acids such as sodium citrate or sodium lactate, organic bases such as arginine or ethanolamine can be used. If necessary, a preservative and/or an antioxidant can be added.

For example, when a solid preparation for oral administration is prepared, filler and, if necessary, binder, disintegrating agent, lubricant, coloring agent and/or flavoring agent are added to the main ingredient, followed by subjecting to a common method to make into tablets, coated tablets, granules, fine granules, powders or capsules.

Examples of the filler are lactose, corn starch, sucrose, glucose, sorbitol, crystalline cellulose and silicon dioxide; examples of the binder are polyvinyl alcohol, ethyl cellulose, methyl cellulose, gum arabic, hydroxypropyl cellulose and hydroxypropyl methyl cellulose; examples of the lubricant are magnesium stearate, talc and silica; examples of the coloring agent are those which are allowed to add to pharmaceuticals; and examples of the flavoring agents are cacao powder, menthol, aromatic, peppermint oil, borneol, and cinnamon powder. It is of course no problem that such tablets and granules are appropriately coated with a sugar coat, gelatin coat or others if necessary.

In preparing injections, a pH adjusting agent, a buffer, a suspending agent, a solubilizer, a stabilizer, an isotonizing agent, a preservative etc. are added, if necessary, to the main ingredient, followed by subjecting to a conventional method to make into injections for intravenous, subcutaneous or intramuscular administration. At that time, it may be made into a freeze-dried product by a common method if necessary.

Examples of the suspending agent are methyl cellulose, polysorbate 80, hydroxyethyl cellulose, gum arabic, tragacanth powder, sodium carboxymethyl cellulose and polyoxyethylene sorbitan monolaurate.

Examples of the solubilizer are polyoxyethylene hydrogenated castor oil, polysorbate 80, nicotinamide, polyoxyethylene sorbitan monolaurate, macrogol and castor oil fatty acid ethyl ester.

Examples of the stabilizer are sodium sulfite and sodium metasulfite. Examples of the preservative are methyl

para-hydroxybenzoate, ethyl para-hydroxybenzoate, sorbic acid, phenol, cresol and chlorocresol.

According to the present invention, there can be provided a novel combined medicinal composition exhibiting an antitumor activity even on cancers against which conventional anticancer drugs are not sufficiently effective. For example, the medicinal composition according to the present invention is useful for treating or preventing brain cancer, head and neck cancer, cancer of the esophagus, cancer of the stomach, cancer of the colon, hepatoma, pancreatic cancer, lung cancer, breast cancer, skin cancer, ovarian cancer, prostatic cancer, renal cancer, bladder cancer, lymphoma and leukemia, Administration of smaller amounts of the combined active ingredients in the composition of the present invention, as compared with administration of large amounts of each single ingredient, hardly causes the side effect of each ingredient, can reduce the side effects of the whole ingredients, and can reduce the cost burden which is caused by using a large amount of an expensive drug for a long time, by simultaneously using inexpensive drugs having a relatively strong effect.

Brief Description of the Drawings

Fig. 1 is a graph showing the synergy of E7070 and CPT-11. The relative tumor volume (RTV) is shown on the ordinate, and the number of days after administration was initiated is shown on the axis;

Fig. 2 is a graph showing the synergy of E7070 and MMC. The relative tumor volume (RTV) is shown on the ordinate, and the number of days after administration was initiated is shown on the axis;

Each of Figs. 3(1), 3(2) and 3(3) is a graph showing the synergy of E7070 and CPT-11. The relative tumor volume (RTV) is shown on the ordinate, and the number of days after administration was initiated is shown on the axis;

Fig. 3(1) shows simultaneous administration of E7070 and CPT-11, Fig. 3(2) shows administration of E7070 and subsequent administration CPT-11, and Fig. 3(3) shows administration of CPT-11 and subsequent administration of E7070;

Fig. 4 is a graph showing the synergy of E7070 and 5-FU. The relative tumor volume (RTV) is shown on the ordinate, and the number of days after administration was initiated is shown on the axis; and

Fig. 5 is a graph showing the synergy of E7070 and CDDP. The relative tumor volume (RTV) is shown on the ordinate, and the number of days after administration was initiated is shown on the axis.

Examples

Hereinafter, Examples and Test Examples are described to show the advantageous effect of the composition of the present invention, but these are illustrative, and in any case the present invention is not limited to the following specific examples.

As described above, the medicinal composition according to the invention, particularly a medicinal composition comprising E7070 or a salt thereof combined with at least one substance selected from (1) irinotecan hydrochloride trihydrate; (2) mitomycin C;(3) 5-fluorouracil; (4) cisplatin; (5) gemcitabine hydrochloride; (6) doxorubicin; (7) taxol; and (8) a salt of the above-mentioned (1) to (7) exhibits an, excellent antitumor activity due to the synergistic antitumor effect of each single drug, and the presence or absence of this synergy was examined according to a two-way ANOVA method (see the following literatures (i) to (iii): (i)Effects of 5-fluorouracil, leucovorin, and glucarate in rat colon-tumor explants. Cancer Chemother Pharmacol. 1992;30(1):25-30; (ii)Enhancement of vincristine cytotoxicity in drug-resistant cells by simultaneous treatment with onconase, an antitumor ribonuclease. J Natl Cancer Inst. 1996 Jun 5;88(11):747-53; (iii) Effects of growth hormone and testosterone on cortical bone formation and bone density in aged orchiectomized rats. Bone. 1999 May;24(5):491-7).

Example 1 Combined use of E7070 and CPT-11 in human colon cancer HCT15 xenograft model

Human colon cancer strain HCT15 (purchased from ATCC) was cultured in RPMI1640 (containing 10 % FBS) in a 5 % $CO_2$ gas incubator until it attained about 80% confluence, and the cells were harvested with trypsin-EDTA and suspended in Hanks balanced solution to prepare a suspension of $5 \times 10^7$ cells/ml. 0.1 ml of the cell suspension was implanted subcutaneously in each nude mouse. When the average tumor volume reached 182 $mm^3$ after the implantation, E7070 in a dose of 30 mg/kg/day and/or CPT-11 in a dose of 75 mg/kg/day were administered either alone or in combination. E7070 was intravenously administered once per day for 5 days (first to fifth days), while CPT-11 was intravenously administered 3 times (once every 4 days, that is, on the first, fifth and ninth days) . After the administration was initiated, the longer and shorter diameters of the tumor were measured at the frequency of 2 times/week with digital calipers (Mitsutoyo), and the tumor volume was calculated according to the following equation.

$$\text{Tumor volume} = \text{longer diameter of tumor (mm)} \times \text{shorter diameter of tumor (mm)}^2/2$$

The items for determination of antitumor effect were the following 2 items:

$T_{x4}$: The time in days for tumor size to attain 4 folds of initial size.
Minimum relative tumor volume (mRTV) : Minimum value of relative tumor volume (RTV*)
*: Tumor volume on n days after first treatment/tumor volume at the start of treatment.

When the group treated with the combined drugs exhibited an antitumor effect superior to that of the group treated with the single drug and simultaneously a statistically significant interaction was recognized in two-way ANOVA, the antitumor effect was determined to be synergistic. Results are as shown in Table 1 and Fig. 1.

As is evident from Table 1 and Fig. 1, combined use of E7070 and CPT-11 permits their respective effects to be synergistically increased, indicating that a combined drug of E7070 and CPT-11 serves as an excellent anticancer agent.

Example 2 Combined use of E7070 and MMC in human colon cancer HCT15 xenograft model

Human colon cancer strain HCT15 (purchased from ATCC) was cultured in RPMI1640 (containing 10 % FBS) in a 5 % $CO_2$ gas incubator until it attained about 80% confluence, and the cells were harvested with trypsin-EDTA and suspended in Hanks balanced solution to prepare a suspension of $5 \times 10^7$ cells/ml. 0.1 ml of the cell suspension was implanted subcutaneously in each nude mouse. When the average tumor volume reached 156 $mm^3$ after the implantation, E7070 in a dose of 25 mg/kg/day and/or MMC in a dose of 4.19 mg/kg/day were administered either alone or in combination. E7070 was intravenously administered once per day for 5 days (first to fifth days), while MMC was intravenously administered once (first day). After the administration was initiated, the longer and shorter diameters of the tumor were measured at the frequency of 2 times/week with digital calipers (Mitsutoyo), and the tumor volume was calculated according to the following equation.

$$\text{Tumor volume} = \text{longer diameter of tumor (mm)} \times \text{shorter diameter of tumor (mm)}^2/2$$

The items for determination of antitumor effect were the following 2 items:

$T_{x4}$: The time in days for tumor size to attain 4 folds of initial size.
Minimum relative tumor volume (mRTV) : Minimum value of relative tumor volume (RTV*)
*: Tumor volume on n days after first treatment/tumor volume at the start of treatment.

When the group treated with the combined drugs exhibited an antitumor effect superior to that of the group treated with the single drug and simultaneously a statistically significant interaction was recognized in two-way ANOVA, the antitumor effect was determined to be synergistic. Results are as shown in Table 2 and Fig. 2.

As is evident from Table 2 and Fig. 2, combined use of E7070 and MMC permits their respective effects to be synergistically increased, indicating that a combined drug of E7070 and MMC serves as an excellent anticancer agent.

Example 3 Combined use of E7070 and CPT-11 in human colon cancer SW620 xenograft model

Human colon cancer strain SW620 (purchased from ATCC) was cultured in RPMI1640 (containing 10 % FBS) in a 5 % $CO_2$ gas incubator until it attained about 80% confluence, and the cells were harvested with trypsin-EDTA and suspended in Hanks balanced solution to prepare a suspension of $5 \times 10^7$ cells/ml. 0.1 ml of the cell suspension was implanted subcutaneously in each nude mouse. When the average tumor volume reached 226 $mm^3$ after the implantation, E7070 in a dose of 25 mg/kg/day and/or CPT-11 in a dose of 62.5 mg/kg/day were administered either alone or in combination. E7070 alone was intravenously administered once per day for 5 days (first to fifth days), while CPT-11 alone was intravenously administered 3 times (once every 4 days, that is, on the first, fifth and ninth days). Administration in combination was performed in 3 schedules, that is, simultaneous administration (E7070 on the first to fifth days; CPT-11 on the first, fifth and ninth days), previous administration of E7070 (E7070 on the first to fifth days; CPT-11 on the sixth, tenth and fourteenth days) and previous administration of CPT-11 (E7070 on the tenth to fourteenth days; CPT-11 on the first, fifth and ninth days). After the administration was initiated, the longer and shorter diameters of the tumor were measured at the frequency of 2 times/week with digital calipers (Mitsutoyo), and the tumor volume was calculated according to the following equation.

$$\text{Tumor volume} = \text{longer diameter of tumor (mm)} \times \text{shorter diameter of tumor (mm)}^2/2$$

The items for determination of antitumor effect were the following 2 items:

$T_{x4}$ : The time in days for tumor size to attain 4 folds of initial size.
Minimum relative tumor volume (mRTV) : Minimum value of relative tumor volume (RTV*)
*: Tumor volume on n days after first treatment/tumor volume at the start of treatment.

When the group treated with the combined drugs exhibited an antitumor effect superior to that of the group treated with the single drug and simultaneously a statistically significant interaction was recognized in two-way ANOVA, the antitumor effect was determined to be synergistic. Results are as shown in Table 3 and Fig. 3.

As is evident from Table 3 and Fig. 3, combined use of E7070 and CPT-11 permits their respective effects to be synergistically increased, indicating that a combined drug of E7070 and CPT-11 serves as an excellent anticancer drug. Further, E7070 and CPT-11 may be administered simultaneously, or one of the two may be administered after a predetermined time, and E7070 and CPT-11, whichever is administered first, exhibits a synergistic effect.

Example 4 Combined use of E7070 and 5-FU in human colon cancer Colo320D.M. xenograft model

Human colon cancer strain Colo320D.M. (purchased fromATCC) was cultured in RPMI1640 (containing 10 % FBS) in a 5 % $CO_2$ gas incubator until it attained about 80% confluence, and the cells were harvested with trypsin-EDTA and suspended in Hanks balanced solution to prepare a suspension of $8\times10^7$ cells/ml. 0.1 ml of the cell suspension was implanted subcutaneously in each nude mouse. When the average tumor volume reached 169 $mm^3$ after the implantation, E7070 in a dose of 30 mg/kg/day and/or 5-FU in a dose of 60 mg/kg/day were administered either alone or in combination. E7070 alone was intravenously administered per day for 5 days (first to fifth days), while 5-FU alone was intravenously administered 3 times (once every 4 days, that is, on the first, fifth and ninth days). Administration in combination was performed in 3 schedules, that is, simultaneous administration (E7070 on the first to fifth days; 5-FU on the first, fifth and ninth days), previous administration of E7070 (E7070 on the first to fifth days; 5-FU on the sixth, tenth and fourteenth days) and previous administration of 5-FU (E7070 on the tenth to fourteenth days; 5-FU on the first, firth and ninth days) . After the administration was initiated, the longer and shorter diameters of the tumor were measured at the frequency of 2 times/week with digital calipers (Mitsutoyo), and the tumor volume was calculated according to the following equation.

$$\text{Tumor volume} = \text{longer diameter of tumor (mm)} \times \text{shorter diameter of tumor (mm)}^2/2$$

The items for determination of antitumor effect were the following 2 items:

$T_{x4}$: The time in days for tumor size to attain 4 folds of initial size.
Minimum relative tumor volume (mRTV) : Minimum value of relative tumor volume (RTV*)
*: Tumor volume on n days after first treatment/tumor volume at the start of treatment.

When the group treated with the combined drugs exhibited an antitumor effect superior to that of the group treated with the single drug and simultaneously a statistically significant interaction was recognized in two-way ANOVA, the antitumor effect was determined to be synergistic. Results are as shown in Table 4 and Fig. 4.

As is evident from Table 4 and Fig. 4, combined use of E7070 and 5-FU permits their respective effects to be

synergistically increased, indicating that a combined drug of E7070 and 5-FU serves as an excellent anticancer agent.

Example 5 Combined use of E7070 and CDDP in human non-small cell lung cancer LU-99 xenograft model

Human non-small cell lung cancer LU-99 (purchased from Human Science Research Resources Bank) was cultured in RPMI1640 (containing 10 % FBS) in a 5 % $CO_2$ gas incubator until it attained about 80% confluence, and the cells were harvested with trypsin-EDTA and suspended in Hanks balanced solution to prepare a suspension of $6.4\times10^7$ cells/ml. 0.1 ml of the cell suspension was implanted subcutaneously in each nude mouse. When the average tumor volume reached 114 $mm^3$ after the implantation, E7070 in a dose of 25 mg/kg/day and/or CDDP in a dose of 7.5 mg/kg/day were administered either alone or in combination. E7070 alone was intravenously administered once per day for 5 days (first to fifth days), while CDDP alone was intravenously administered once (first day). Administration in combination was performed in 3 schedules, that is, simultaneous administration (E7070 on the first to fifth days; CDDP on the first day), previous administration of E7070 (E7070 on the first to fifth days; CDDP on the sixth day) and previous administration of CDDP (E7070 on the second to sixth days; CDDP on the first day). After the administration was initiated, the longer and shorter diameters of the tumor were measured at the frequency of 2 times/week with digital calipers (Mitsutoyo), and the tumor volume was calculated according to the following equation.

$$\text{Tumor volume} = \text{longer diameter of tumor (mm)} \times \text{shorter diameter of tumor (mm)}^2/2$$

The items for determination of antitumor effect were the following 2 items:

$T_{\times4}$: The time in days for tumor size to attain 4 folds of initial size.
Minimum relative tumor volume (mRTV) : Minimum value of relative tumor volume (RTV*)
*: Tumor volume on n days after first treatment/tumor volume at the start of treatment.

When the group treated with the combined drugs exhibited an antitumor effect superior to that of the group treated with the single drug and simultaneously a statistically significant interaction was recognized in two-way ANOVA, the antitumor effect was determined to be synergistic. Results are as shown in Table 5 and Fig. 5.

As is evident from Table 5 and Fig. 5, combined use of E7070 and CDDP permits their respective effects to be synergistically increased, indicating that a combined drug of E7070 and CDDP serves as an excellent anticancer agent.

Example 6 Evaluation of the effect of combined use of E7070 and gemcitabine in human colon cancer HCT15 xenograft model

Human colon cancer strain HCT15 (purchased from ATCC) was cultured in RPMI1640 (containing 10 % FBS) in a 5 % $CO_2$ gas incubator until it attained about 80% confluence, and the cells were harvested with trypsin-EDTA and suspended in Hanks balanced solution to prepare a suspension of $5\times10^7$ cells/ml. 0.1 ml of the cell suspension was implanted subcutaneously in each nude mouse. When the average tumor volume reached 156 $mm^3$ after the implantation, E7070 in a dose of 25 mg/kg/day and/or MMC in a dose of 4.19 mg/kg/day were administered either alone or in combination. E7070 was intravenously administered once per day for 5 days (first to fifth days), while CPT-11 was intravenously administered 4 times (once every 3 days, that is, on the first, fourth, seventh and tenth days). After the administration was initiated, the longer and shorter diameters of the tumor were measured at the frequency of 2 times/week with digital calipers (Mitsutoyo), and the tumor volume was calculated according to the following equation.

$$\text{Tumor volume} = \text{longer diameter of tumor (mm)} \times \text{shorter diameter of tumor (mm)}^2/2$$

The items for determination of antitumor effect were the following 2 items:

$T_{x4}$: The time in days for tumor size to attain 4 folds of initial size.
Minimum relative tumor volume (mRTV) : Minimum value of relative tumor volume (RTV*)

*: Tumor volume on n days after first treatment/tumor volume at the start of treatment.

When the group treated with the combined drugs exhibited an antitumor effect superior to that of the group treated with the single drug and simultaneously a statistically significant interaction was recognized in two-way ANOVA, the antitumor effect was determined to be synergistic.

As is evident from the above-mentioned experimental examples, the compositions of the present invention exhibit an excellent antitumor activity and are useful as an antitumor agent. In addition to the compounds used above, gemcitabine hydrochloride, doxorubicin and taxol were used and examined for their synergistic effect, indicating that they also exhibit an excellent antitumor activity and are useful as an antitumor agent. As the cancer type, cancers against which the respective ingredients are effective may be proposed, and since it varies depending on the constitution of the composition and further the synergistic effect in the present invention is higher than that of a single drug, the type of cancer is not particularly limited.

Table 1

Antitumor effect of single or combined drug of E7070 and CPT-11 in HCT15 xenograft model

| Treatment | | $T_{x4}$ (days)[a] | | mRTV[b] | | mRBW[c] | Toxicity |
|---|---|---|---|---|---|---|---|
| | | mean±S.D. | Two way ANOVA | mean±S.D. | Two way ANOVA | mean | Dead /Treated |
| Control | | 9.5±1.8 | - | 4.41±1.02(10)[f] | - | 0.97(7, 25) | 0/6 |
| E7070[d] | 30mg/kg/day | 18.7±2.0 | - | - | - | 0.92(7) | 0/6 |
| CPT-11[e] | 75 mg/kg/day | 17.8±1.0 | - | - | - | 0.94(7, 10) | 0/6 |
| E7070 30 mg/kg/day + CPT-11 75 mg/kg/day | | 31.7±4.2 | $P<0.05$ Synergistic | 0.83±0.17(10) | $P<0.05$ Synergistic | 0.85(7) | 0/6 |

a): $T_{x4}$: The time in days for tumor size to attain 4 folds of initial tumor size.
b): m(minimum)RTV: RTV= tumor volume on n days after first treatment / tumor volume at the start of treatment, The parentheses indicated the day when RTV was minimal. In control group, RTV on day 10 is indicated.
c): m(minimum)RBW: RBW= body weight on n days after first treatment / body weight at the start of treatment The parentheses indicated the day when RBW was minimal.
d): QDx5 daily for 5 days administration (day 1, 2, 3, 4, 5)
e): Q4Dx3 every 4 days, 3 times administration (day 1, 5, 9)
f) RTV of control group on day 10

Table 2

Antitumor effect of single or combined drug of E7070 and MMC in HCT15 xenograft model

| Treatment | $T_{x4}$ (days)[a] mean±S.D. | $T_{x4}$ (days)[a] Two way ANOVA | mRTV[b] mean±S.D. | mRTV[b] Two way ANOVA | mRBW[c] mean | Toxicity Dead /Treated |
|---|---|---|---|---|---|---|
| Control | 8.7±1.9 | - | - | - | 0.99(11) | 0/6 |
| E7070[d] 25mg/kg/day | 12.7±2.2 | - | - | - | 0.93(11) | 0/6 |
| MMC[e] 4.19 mg/kg/day | 16.2±3.4 | - | - | - | 0.99(11) | 0/6 |
| E7070 30 mg/kg/day + MMC 4.19 mg/kg/day | 26.5±5.3 | P<0.05 Synergistic | - | - | 0.93(11) | 0/6 |

a): $T_{x4}$; The time in days for tumor size to attain 4 folds of initial tumor size.
b): m(minimum)RTV: RTV= tumor volume on n days after first treatment / tumor volume at the start of treatment,
In all dose described, tumor regression was not observed.
c): m(minimum)RBW: RBW= body weight on n days after first treatment / body weight at the start of treatment
The parentheses indicated the day when RBW was minimal.
d): QDx5 daily for 5 days administration (day 1, 2, 3, 4, 5)
e): QDx1 single dose (day 1)

Table 3

Antitumor effect of single or combined drug of E7070 and CPT-11 in SW620 xenograft model

| Treatment | $T_{x4}$ (days) [a)] mean±S.D. | $T_{x4}$ (days) [a)] Two way ANOVA | mRTV [b)] mean±S.D. | mRTV [b)] Two way ANOVA | mRBW [c)] mean | Toxicity Dead /Treated |
|---|---|---|---|---|---|---|
| Control | 9.0±2.2 | - | 6.28±4.62(21) | - | 0.90(18) | 0/6 |
| E7070 [d)] 25mg/kg/day | 30.2±6.1 | - | 0.76±0.42(12) | - | 0.98(8) | 0/6 |
| CPT-11 [e)] 62.5 mg/kg/day | 48.7±29.7 | - | - | - | 0.99(8) | 0/6 |
| E7070 25 mg/kg/day [d)] + CPT-11 62.5 mg/kg/day [e)] | 60.0±8.4 | P>0.05 additive | 0.15±0.02(21) | P<0.05 Synergistic | 0.86(8) | 0/6 |
| E7070 25 mg/kg/day [d)] → CPT-11 62.5 mg/kg/day [f)] | 65.2±8.5 | P>0.05 additive | 0.17±0.09(21) | P<0.05 Synergistic | 0.90(8) | 0/6 |
| CPT-11 62.5 mg/kg/day [e)] → E7070 25 mg/kg/day [g)] | 62.2±10.5 | P>0.05 additive | 0.26±0.08(21) | P<0.05 Synergistic | 0.95(15) | 0/6 |

a): $T_{x4}$: The time in days for tumor size to attain 4 folds of initial tumor size.
b): m(minimum)RTV: RTV= tumor volume on n days after first treatment / tumor volume at the start of treatment,
The parentheses indicated the day when RTV was minimal. In control group, RTV on day 21 is indicated.
c): m(minimum)RBW: RBW= body weight on n days after first treatment / body weight at the start of treatment
The parentheses indicated the day when RBW was minimal.
d): QDx5 daily for 5 days administration (day 1, 2, 3, 4, 5)
e): Q4Dx3 every 4 days, 3 times administration (day 1, 5, 9)
f) : Q4Dx3 every 4 days, 3 times administration (day 6, 10, 14)
g): QDx5 daily for 5 days administration (day 10, 11, 12, 13, 14)

Table 4

Antitumor effect of single or combined drug of E7070 and 5-FU in Colo320D.M xenograft model

| Treatment | $T_{x4}$ (days)[a)] mean±S.D. | $T_{x4}$ (days)[a)] Two way ANOVA | mRTV[b)] mean±S.D. | mRTV[b)] Two way ANOVA | mRBW[c)] mean | Toxicity Dead /Treated |
|---|---|---|---|---|---|---|
| Control | 6.0±0.7 | - | - | - | - | 0/6 |
| E7070[d)] 30mg/kg/day | 11.2±1.9 | - | - | - | - | 0/6 |
| 5-FU[e)] 60 mg/kg/day | 10.0±0.7 | - | - | - | - | 0/6 |
| E7070 30 mg/kg/day[d)] + 5-FU 60 mg/kg/day[e)] | 26.0±2.1 | P<0.05 Synergistic | - | - | 0.91(8) | 0/6 |
| E7070 30 mg/kg/day[d)] → 5-FU 60 mg/kg/day[f)] | 15.2±2.6 | p>0.05 additive | - | - | 0.99(8) | 0/6 |
| 5-FU 60 mg/kg/day[e)] → E7070 30 mg/kg/day[g)] | 8.6±0.89 | p>0.05 additive | - | - | 0.99(15) | 0/6 |

a): $T_{x4}$: The time in days for tumor size to attain 4 folds of initial tumor size.
b): m(minimum)RTV: RTV= tumor volume on n days after first treatment / tumor volume at the start of treatment,
"-": Tumor regression was not observed.
c): m(minimum)RBW: RBW= body weight on n days after first treatment / body weight at the start of treatment
The parentheses indicated the day when RBW was minimal. "-": body weight loss was not observed.
d): QDx5 daily for 5 days administration (day 1, 2, 3, 4, 5)
e): Q4Dx3 every 4 days, 3 times administration (day 1, 5, 9)
f) : Q4Dx3 every 4 days, 3 times administration (day 6, 10, 14)
g): QDx5 daily for 5 days administration (day 10, 11, 12, 13, 14)

Table 5

Antitumor effect of single or combined drug of E7070 and CDDP in LU-99 xenograft model

| Treatment | $T_{x4}$ (days)[a] mean±S.D. | $T_{x4}$ (days)[a] Two way ANOVA | mRTV[b] mean±S.D. | mRTV[b] Two way ANOVA | mRBW[c] mean | Toxicity Dead /Treated |
|---|---|---|---|---|---|---|
| Control | 8.3±1.2 | - | - | - | - | 0/6 |
| E7070[d] 25mg/kg/day | 18.2±2.3 | - | 0.75±0.16 | - | - | 0/6 |
| CDDP[e] 7.5 mg/kg/day | 10.7±0.5 | - | - | - | 0.95(5) | 0/6 |
| E7070 25 mg/kg/day[d] + CDDP 7.5 mg/kg/day[e] | 26.0±5.1 | P<0.05 Synergistic | 0.97±0.21 | P>0.05 Additive | 0.82(8) | 0/6 |
| E7070 25 mg/kg/day[d] → CDDP 7.5 mg/kg/day[f] | 28.0±8.2 | P<0.05 Synergistic | 0.82±0.22 | P>0.05 Additive | 0.86(8) | 0/6 |
| CDDP 7.5 mg/kg/day[e] → E7070 25 mg/kg/day[g] | 20.3±2.2 | P>0.05 Additive | 0.99±0.21 | P>0.05 Additive | 0.88(8) | 0/6 |

a): $T_{x4}$: The time in days for tumor size to attain 4 folds of initial tumor size.
b): m(minimum)RTV: RTV= tumor volume on n days after first treatment / tumor volume at the start of treatment,
"-": Tumor regression was not observed.
c): m(minimum)RBW: RBW= body weight on n days after first treatment / body weight at the start of treatment
The parentheses indicated the day when RBW was minimal.
d): QDx5 daily for 5 days administration (day 1, 2, 3, 4, 5)
e): QDx1 single dose(day 1)
f) : QDx1 single dose(day 6)
g): QDx5 daily for 5 days administration (day 2, 3, 4, 5, 6)

## Claims

**1.** Pharmaceutical composition comprising

i) a compound of formula (I) or a salt thereof

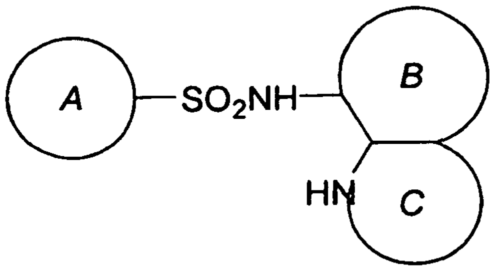

(I)

wherein

ring *A* is benzene or pyridine each optionally substituted with 1-3 groups independently selected from amino, ($C_{1-6}$-alkyl)amino, ($C_{3-8}$-cycloalkyl)amino, $C_{1-6}$-alkyl, $C_{1-6}$-alkoxy, hydroxyl, nitro, mercapto, cyano, $C_{1-6}$-alkylthio, halogen, and groups selected from the formulae (i) - (vi) :

(i) -a-$CH_2$-d
(ii) -a-e-f
(iii) -a-g-h
(iv) -a-N($R^6$)-g-i
(v) -a-N($R^7$)-e-f
(vi) -$(CH_2)_p$-j-$(CH_2)_q$-d

wherein

a is a single bond or -Z-$(CH_2)_k$-;
d is amino, ($C_{1-6}$-alkyl)amino, halogen, hydroxyl, $C_{1-6}$-alkylthio, cyano or $C_{1-6}$-alkoxy;
e is -SO- or -$SO_2$-;
f is amino, ($C_{1-6}$-alkyl)amino, ($C_{2-6}$-alkoxy)amino, $C_{1-6}$-alkyl, trifluoromethyl, -$(CH_2)_m$-d or -N($R^4$)-$(CH_2)_m$-d;
g is -CO- or -CS-;
h is amino, hydroxyl, $C_{1-6}$-alkyl, $C_{1-6}$alkoxy, -$(CH_2)_n$-d or -N($R^5$)-$(CH_2)_n$-d (wherein d is as defined above);
i is H, $C_{1-6}$-alkoxy or f as defined above;
j is O or S;
Z is a bond, O S or $NR^3$;

$R^3$, $R^4$, $R^5$, $R^6$ and $R^7$ are each H or $C_{1-6}$-alkyl; k and p are each an integer of 1-5; and m, n and q are each an integer of 2-6;
ring *B* is optionally substituted benzene; and
ring *C* is optionally substituted pyrrole; rings *B* and *C* each individually may be substituted with 1-2 groups independently selected from halogen, cyano, $C_{1-6}$-alkyl, $C_{1-6}$-alkoxy, hydroxyl, oxo, amino, ($C_{1-6}$-alkyl) amino, trifluoromethyl, and -CO-r (wherein r is selected from H, amino, ($C_{1-6}$-alkyl)amino, $C_{1-6}$-alkyl, $C_{1-6}$-alkoxy and hydroxyl)

and
ii) at least one compound (III) selected from irinotecan hydrochloride trihydrate, cisplatin; mitomycin C, 5-fluorouracil; gemcitabine hydrochloride; taxol; doxorubicin; and salts thereof.

**2.** Pharmaceutical composition of claim 1, comprising, as a compound of formula (I), N-(3-chloro-1H-indol-7-yl)-4-sulfamoylbenzenesulfonamide or a salt thereof.

3. Pharmaceutical composition of claim 2, which comprises, as a compound (III), irinotecan hydrochloride trihydrate or a salt thereof.

4. Pharmaceutical composition of claim 2, which comprises, as a compound (III), mitomycin C or a salt thereof.

5. Pharmaceutical composition of claim 2, which comprises, as a compound (III), 5-fluorouracil or a salt thereof.

6. Pharmaceutical composition of claim 2, which comprises, as a compound (III), cisplatin or a salt thereof.

7. Pharmaceutical composition of claim 2, which comprises, as a compound (III), irinotecan hydrochloride trihydrate or a salt thereof, and cisplatin or a salt thereof.

8. Pharmaceutical composition of claim 7, which further comprises 5-fluorouracil or a salt thereof.

9. Pharmaceutical composition of any of the preceding claims wherein the sulfone compound (I) and the substance (III) are present for simultaneous or separate administration to a patient.

10. Use of a combination of

i) a compound of formula (I) or a salt thereof as defined in claim 1, and
ii) at least one compound (III) selected from irinotecan hydrochloride trihydrate; mitomycin C; cisplatin; 5-fluorouracil; gemcitabine hydrochloride; taxol; doxorubicin; and salts thereof,

for producing a medicament effective in the prevention or treatment of cancer.

11. Use of claim 10, wherein the compound of formula (I) is N-(3-chloro-1H-indol-7-yl)-4-sulfamoylbenzenesulfonamide or a salt thereof.

12. Use of claim 10 or 11, wherein the medicament is for simultaneous or separate administration of the sulfone compound (I) and the substance (III) to a patient.

## Patentansprüche

1. Pharmazeutische Zusammensetzung, die folgendes umfasst:

i) eine Verbindung der Formel (I) oder ein Salz davon

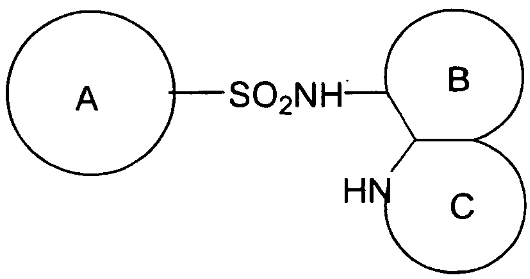

(I)

worin

Ring A Benzol oder Pyridin ist, jeweils ggf. substituiert mit 1-3 Gruppen, die unabhängig voneinander ausgewählt sind aus Amino, ($C_{1-6}$-Alkyl)amino, ($C_{3-8}$-Cycloalkyl)amino, $C_{1-6}$-Alkyl, $C_{1-6}$-Alkoxy, Hydroxyl, Nitro, Mercapto, Cyano, $C_{1-6}$-Alkylthio, Halogen und Gruppen, die aus den Formeln (i)-(vi) ausgewählt sind:

(i) -a-$CH_2$-d

(ii) -a-e-f
(iii) -a-g-h
(iv) -a-N($R^6$)-g-i
(v) -a-N($R^7$)-e-f
(vi) -$(CH_2)_p$-j-$(CH_2)_q$-d

worin

a eine Einfachbindung oder -Z-$(CH_2)_k$- ist;
d Amino, ($C_{1-6}$-Alkyl)amino, Halogen, Hydroxyl, $C_{1-6}$-Alkylthio, Cyano oder $C_{1-6}$-Alkoxy ist;
e -SO- oder -$SO_2$- ist;
f Amino, ($C_{1-6}$-Alkyl)amino, ($C_{1-6}$-Alkoxy)amino, $C_{1-6}$-Alkyl, Trifluormethyl, -$(CH_2)_m$-d oder -N($R^4$)-$(CH_2)_m$-d ist;
g -CO- oder -CS- ist;
h Amino, Hydroxyl, $C_{1-6}$-Alkyl, $C_{1-6}$-Alkoxy, -$(CH_2)_n$-d oder -N($R^5$)-$(CH_2)_n$-d (worin d wie oben definiert ist) ist;
i H, $C_{1-6}$-Alkoxy oder f wie oben definiert ist;
j O oder S ist;
Z eine Bindung, O, S oder $NR^3$ ist;

$R^3$, $R^4$, $R^5$, $R^6$ und $R^7$ jeweils H oder $C_{1-6}$-Alkyl sind; k und p jeweils eine ganze Zahl von 1-5 darstellen; und m, n und q jeweils eine ganze Zahl von 2-6 darstellen;
Ring B ggf. substituiertes Benzol ist; und
Ring C ggf. substituiertes Pyrol ist;
wobei die Ringe B und C jeweils unabhängig voneinander mit 1-2 Gruppen substituiert sein können, die unabhängig voneinander ausgewählt sind aus Halogen, Cyano, $C_{1-6}$-Alkyl, $C_{1-6}$-Alkoxy, Hydroxyl, Oxo, Amino, ($C_{1-6}$-Alkyl)amino, Trifluormethyl und -CO-r (worin r ausgewählt ist aus H, Amino, ($C_{1-6}$-Alkyl)amino, $C_{1-6}$-Alkyl, $C_{1-6}$-Alkoxy und Hydroxyl);

und
ii) mindestens eine Verbindung (III), die ausgewählt ist aus Irinotecanhydrochlorid-Trihydrat, Cisplatin; Mitomycin C, 5-Fluoruracil; Gemcitabinhydrochlorid; Taxol; Doxorubicin; und Salzen davon.

**2.** Pharmazeutische Zusammensetzung gemäß Anspruch 1, die als Verbindung der Formel (I), N-(3-Chlor-1H-indol-7-yl)-4-sulfamoylbenzolsulfonamid oder ein Salz davon umfasst.

**3.** Pharmazeutische Zusammensetzung gemäß Anspruch 2, die als Verbindung (III) Irinotecanhydrochlorid-Trihydrat oder ein Salz davon umfasst.

**4.** Pharmazeutische Zusammensetzung gemäß Anspruch 2, die als Verbindung (III) Mitomycin C oder ein Salz davon umfasst.

**5.** Pharmazeutische Zusammensetzung gemäß Anspruch 2, die als Verbindung (III) 5-Fluoruracil oder ein Salz davon umfasst.

**6.** Pharmazeutische Zusammensetzung gemäß Anspruch 2, die als Verbindung (III) Cisplatin oder ein Salz davon umfasst.

**7.** Pharmazeutische Zusammensetzung gemäß Anspruch 2, die als Verbindung (III) Irinotecanhydrochlorid-Trihydrat oder ein Salz davon und Cisplatin oder ein Salz davon umfasst.

**8.** Pharmazeutische Zusammensetzung gemäß Anspruch 7, die ferner 5-Fluoruracil oder ein Salz davon umfasst.

**9.** Pharmazeutische Zusammensetzung gemäß mindestens einem der vorhergehenden Ansprüche, worin die Sulfonverbindung (I) und die Substanz (III) zur gleichzeitigen oder getrennten Verabreichung bei einem Patienten vorhanden sind.

**10.** Verwendung einer Kombination von

i) einer Verbindung der Formel (I) oder einem Salz davon wie in Anspruch 1 definiert, und
ii) mindestens einer Verbindung (III) ausgewählt aus Irinotecanhydrochlorid-Trihydrat; Mitomycin C; Cisplatin; 5-Fluoruracil; Gemcitabinhydrochlorid; Taxol; Doxorubicin; und Salzen davon,

zur Herstellung eines Medikaments, das wirksam ist zur Vorbeugung oder Behandlung von Krebs.

**11.** Verwendung gemäß Anspruch 10, worin die Verbindung der Formel (I) N-(3-Chlor-1H-indolyl)-4-sulfamoylbenzolsulfonamid oder ein Salz davon ist.

**12.** Verwendung gemäß Anspruch 10 oder 11, worin das Medikament zur gleichzeitigen oder getrennten Verabreichung der Sulfonverbindung (I) und der Substanz (III) bei einem Patienten vorgesehen ist.

## Revendications

**1.** Composition pharmaceutique comprenant :

i) un composé de formule (I) ou un sel de celui-ci

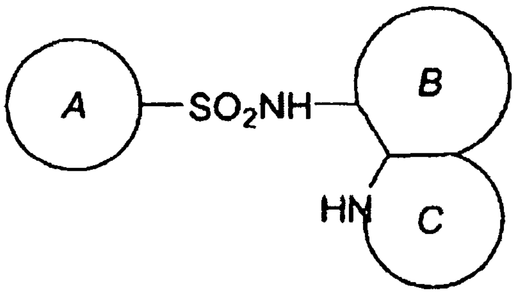

(I)

dans laquelle

le cycle A est un benzène ou une pyridine chacun éventuellement substitué avec 1 - 3 groupes indépendamment choisis parmi un amino, un alkyl (en $C_{1-6}$) amino, un cycloalkyl (en $C_{3-8}$) amino, un alkyle en $C_{1-6}$, un alcoxy en $C_{1-6}$, un hydroxyle, un nitro, un mercapto, un cyano, un alkylthio en $C_{1-6}$, un halogène, et des groupes choisis parmi les formules (i) - (vi) :

(i) $-a-CH_2-d$
(ii) -a-e-f
(iii) -a-g-h
(iv) $-a-N(R^6)-g-i$
(v) $-a-N(R^7)-e-f$
(vi) $-(CH_2)_p-j-(CH_2)_q-d$

dans lesquelles

a est une liaison simple ou $-Z-(CH_2)_k-$;
d est un amino, un alkyl(en $C_{1-6}$)amino, un halogène, un hydroxyle, un alkylthio en $C_{1-6}$, un cyano ou un alcoxy en $C_{1-6}$;
e est -SO- ou $-SO_2-$;
f est un amino, un alkyl (en $C_{1-6}$) amino, un alcoxy(en $C_{1-6}$)amino, un alkyle en $C_{1-6}$, un trifluorométhyle, $-(CH_2)_m-d$ ou $-N(R^4)-(CH_2)_m-d$;
g est -CO- ou -CS-;
h est un amino, un hydroxyle, un alkyle en $C_{1-6}$, un alcoxy en $C_{1-6}$, $-(CH_2)_n-d$ ou $-N(R^5)-(CH_2)_n-d$ (où d est tel que défini ci-dessus);
i est H, un alcoxy en $C_{1-6}$ ou f tel que défini ci-dessus;
j est O ou S;

Z est une liaison, O, S ou $NR^3$;

$R^3$, $R^4$, $R^5$, $R^6$ et $R^7$ sont chacun H ou un alkyle en $C_{1-6}$ ; k et p sont chacun un nombre entier de 1 - 5; et m, n et q sont chacun un nombre entier de 2 - 6;

le cycle B un benzène éventuellement substitué; et
le cycle C est un pyrrole éventuellement substitué;

les cycles B et C chacun individuellement peuvent être substitués avec 1 - 2 groupes indépendamment choisis parmi un halogène, un cyano, un alkyle en $C_{1-6}$, un alcoxy en $C_{1-6}$, un hydroxyle, un oxo, un amino, un alkyl (en $C_{1-6}$) amino, un trifluorométhyle, et -CO-r (où r est choisi parmi H, un amino, un alkyl (en $C_{1-6}$) amino, un alkyle en $C_{1-6}$, un alcoxy en $C_{1-6}$ et un hydroxyle)

et
ii) au moins un composé (III) choisi parmi le chlorhydrate trihydraté d'irinotécan, le cisplatine; la mitomycine C, le 5-fluorouracile; le chlorhydrate de gemcitabine; le taxol; la doxorubicine; et les sels de ceux-ci.

**2.** Composition pharmaceutique selon la revendication 1, comprenant, en tant que composé de formule (I), le N-(3-chloro-1H-indol-7-yl)-4-sulfamoylbenzènesulfonamide ou un sel de celui-ci.

**3.** Composition pharmaceutique selon la revendication 2, qui comprend, en tant que composé (III), du chlorhydrate trihydraté d'irinotécan ou un sel de celui-ci.

**4.** Composition pharmaceutique selon la revendication 2, qui comprend, en tant que composé (III), de la mitomycine C ou un sel de celle-ci.

**5.** Composition pharmaceutique selon la revendication 2, qui comprend, en tant que composé (III), du 5-fluorouracile ou un sel de celui-ci.

**6.** Composition pharmaceutique selon la revendication 2, qui comprend, en tant que composé (III), du cisplatine ou un sel de celui-ci.

**7.** Composition pharmaceutique selon la revendication 2, qui comprend, en tant que composé (III), du chlorhydrate trihydraté d'irinotécan ou un sel de celui-ci, et du cisplatine ou un sel de celui-ci.

**8.** Composition pharmaceutique selon la revendication 7, qui comprend en outre du 5-fluorouracile ou un sel de celui-ci.

**9.** Composition pharmaceutique selon l'une quelconque des revendications précédentes dans laquelle le composé de sulfone (I) et la substance (III) sont présents pour une administration simultanée ou séparée à un patient.

**10.** Utilisation d'une combinaison de

i) un composé de formule (I) ou un sel de celui-ci tel que défini dans la revendication 1, et
ii) au moins un composé (III) choisi parmi le chlorhydrate trihydraté d'irinotécan; la mitomycine C; le cisplatine; le 5-fluorouracile; le chlorhydrate de gemcitabine; le taxol; la doxorubicine; et les sels de ceux-ci,

pour la production d'un médicament efficace dans la prévention ou le traitement d'un cancer.

**11.** Utilisation selon la revendication 10, dans laquelle le composé de formule (I) est le N-(3-chloro-1H-indol-7-yl)-4-sulfamoylbenzènesulfonamide ou un sel de celui-ci.

**12.** Utilisation selon la revendication 10 ou 11, dans laquelle le médicament est pour une administration simultanée ou séparée du composé de sulfone (I) et de la substance (III) à un patient.

Fig. 1

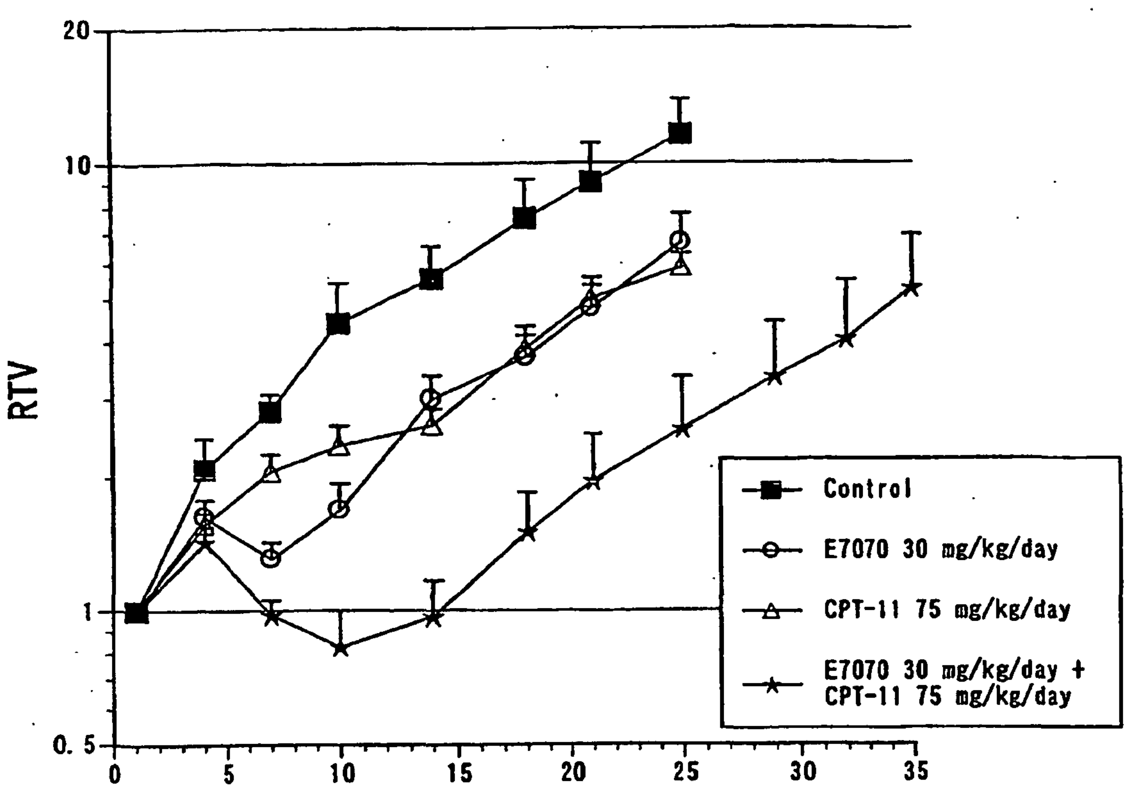


the number of days after administration was initiated

Fig. 2

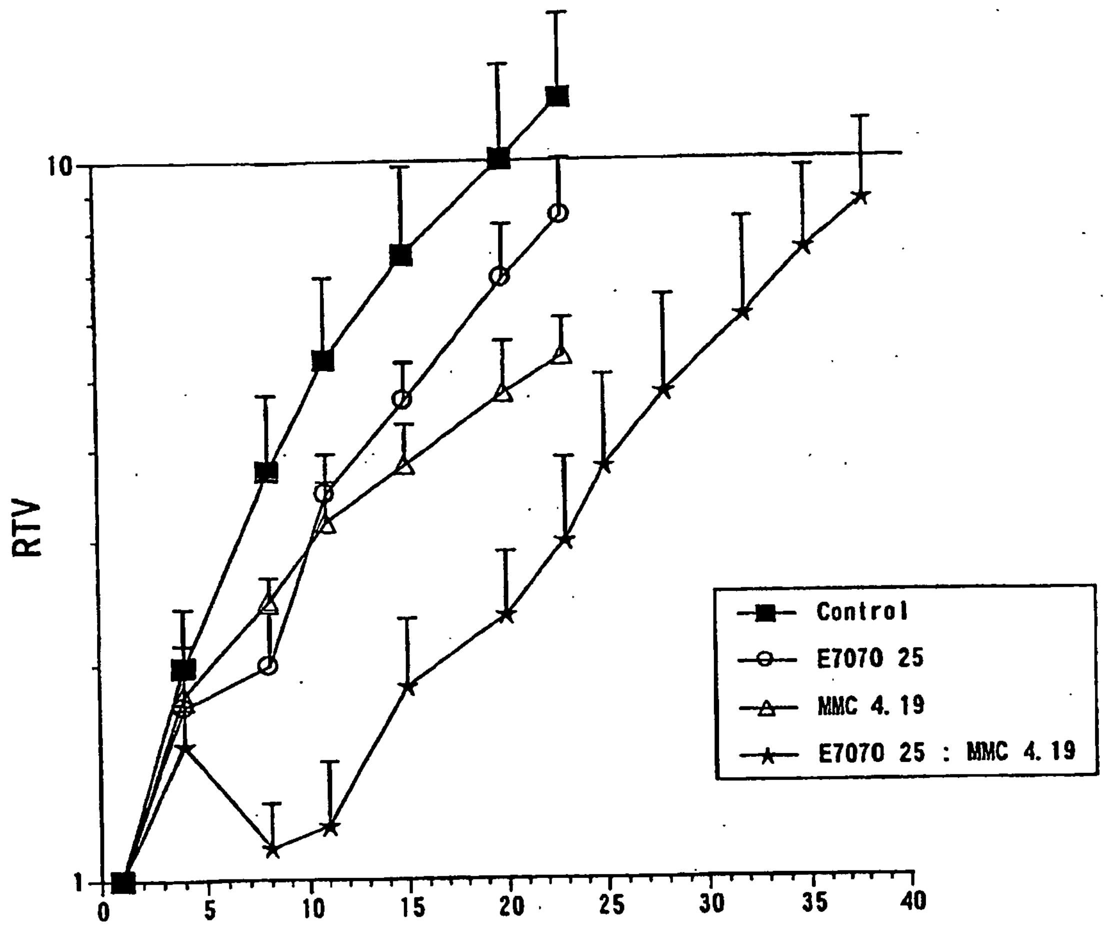


the number of days after administration was initiated

Fig. 3(1)

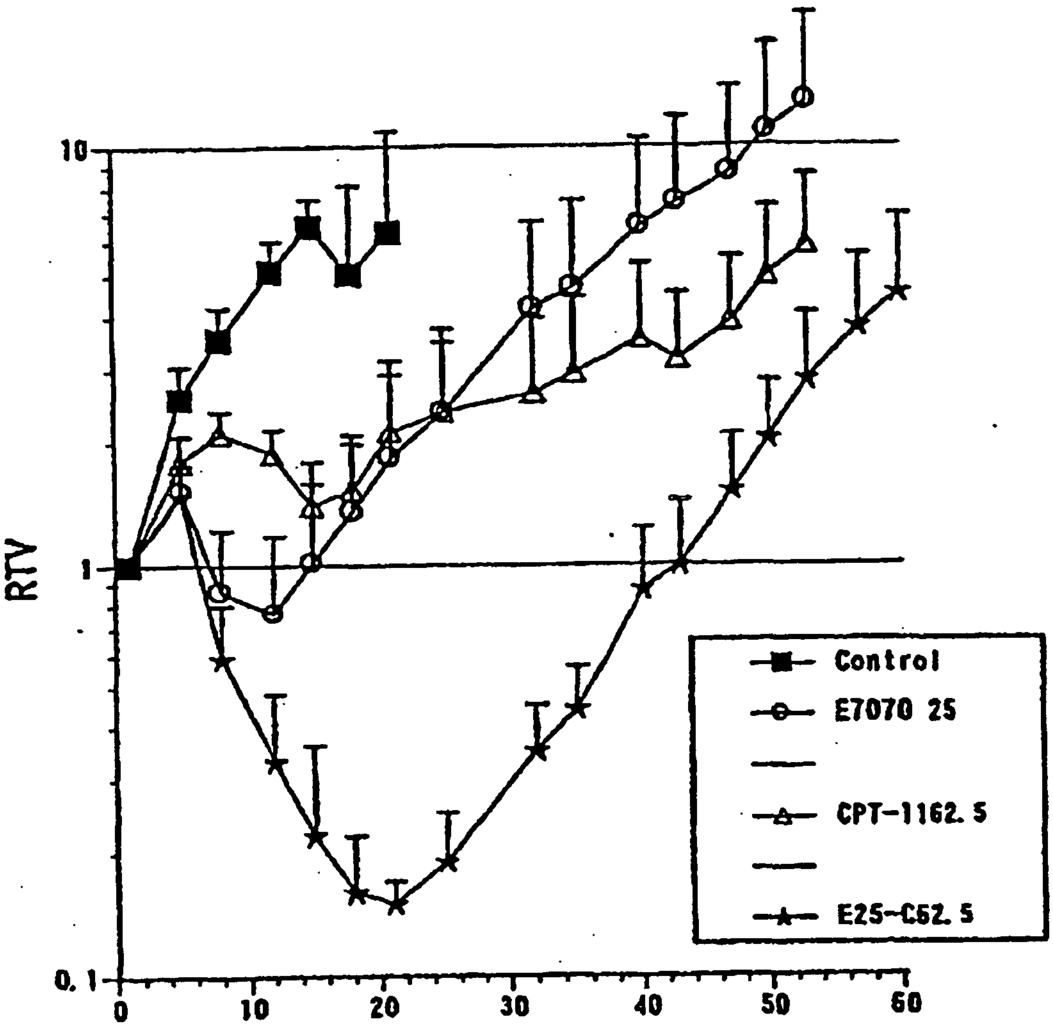


the number of days after administration was initiated

Fig. 3(2)

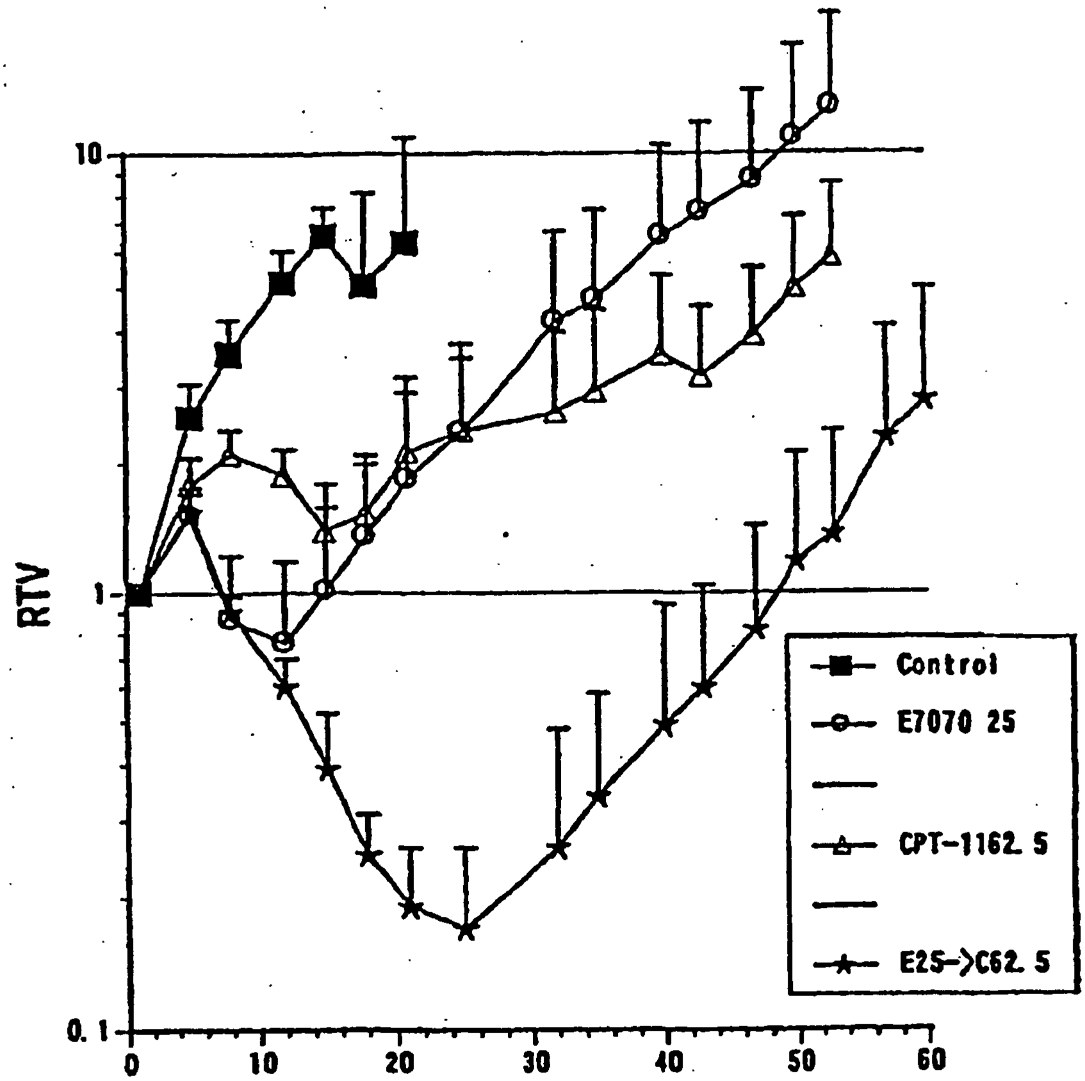


the number of days after administration was initiated

Fig. 3(3)

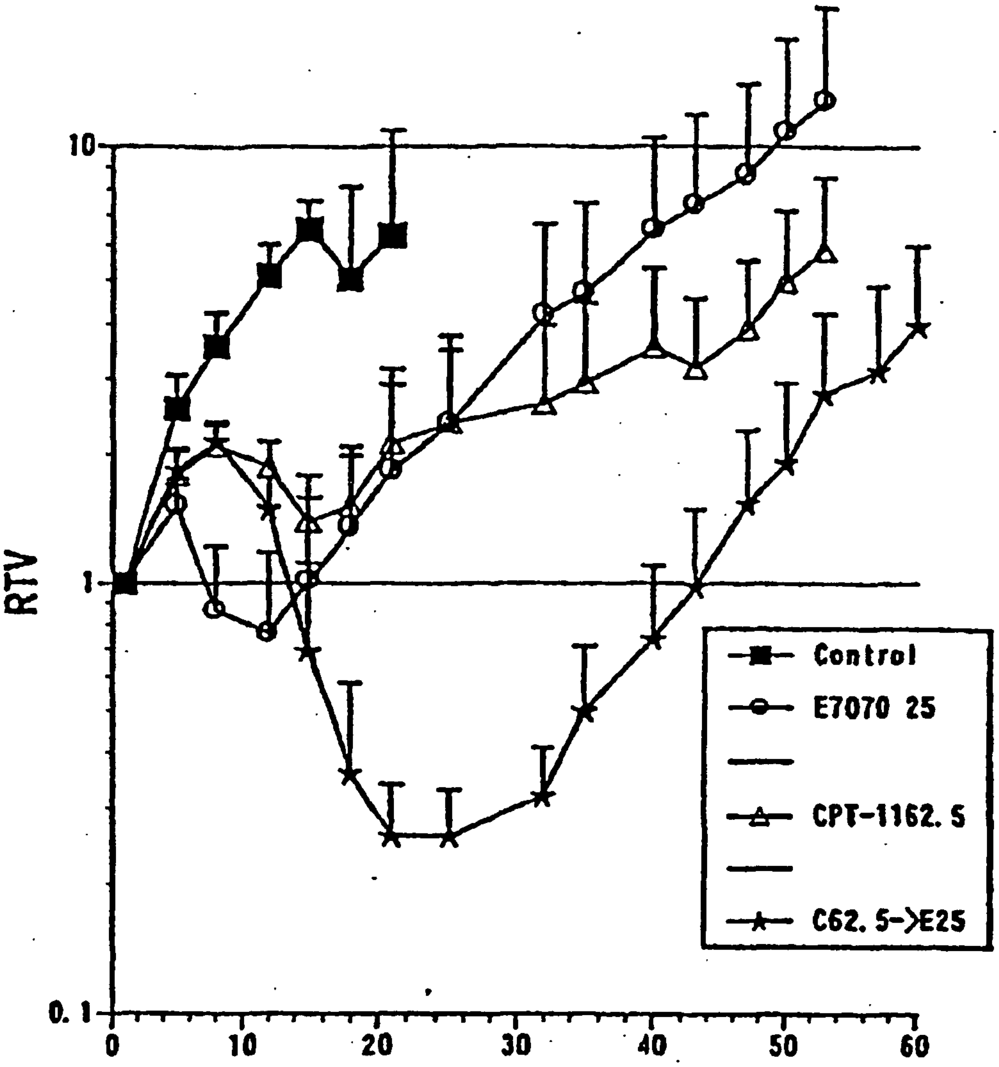


the number of days after administration was initiated

Fig. 4

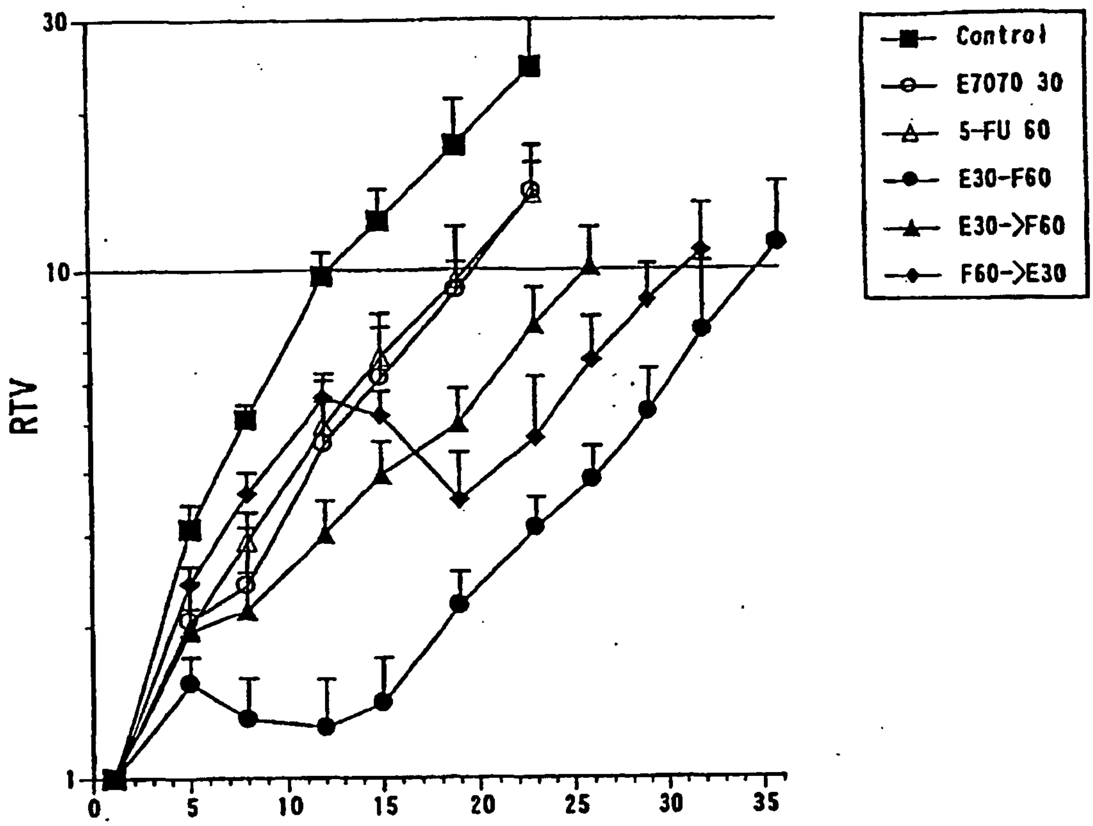


the number of days after administration was initiated

Fig. 5

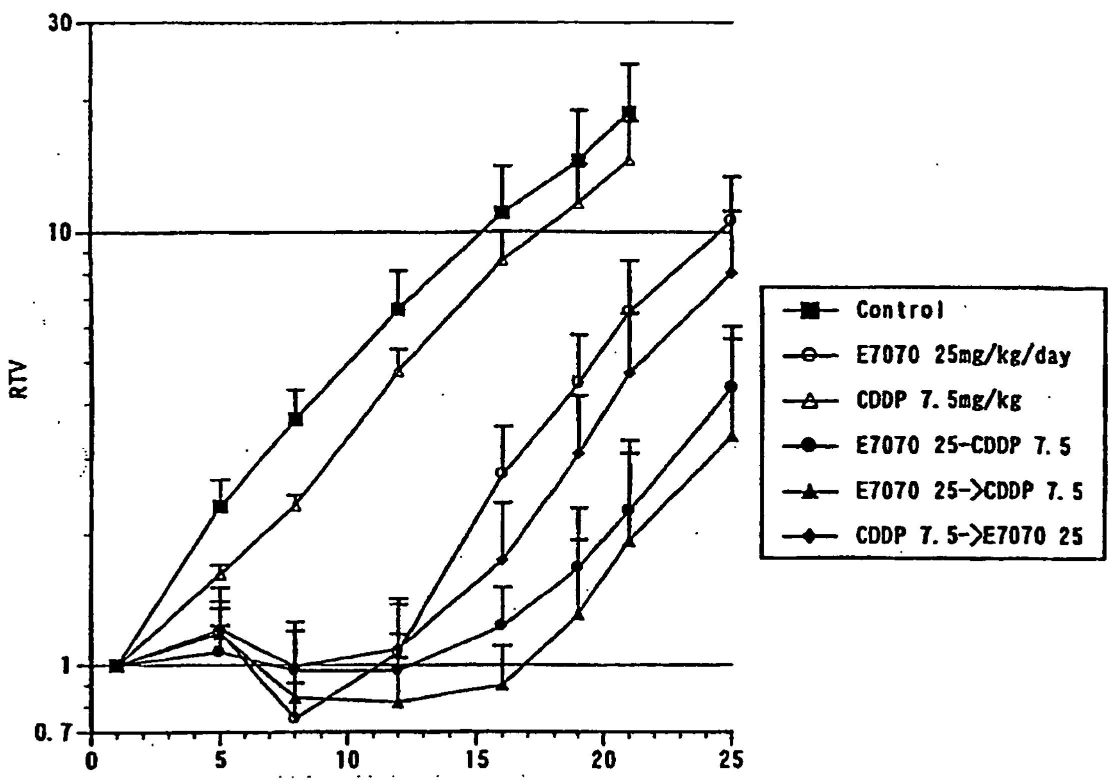


the number of days after administration was initiated